# EUROPEAN PATENT SPECIFICATION

(11) **EP 1 933 895 B1**
(45) Date of publication and mention of the grant of the patent: **11.05.2011**
(21) Application number: 06803037.8
(22) Date of filing: 06.09.2006
(51) Int. Cl.: A61L 31/16

(54) **STENT WITH POCKETS FOR CONTAINING A THERAPEUTIC AGENT**
STENT MIT TASCHEN FÜR EIN THERAPEUTISCHES MITTEL
STENT DOTE DE POCHES DESTINEES A CONTENIR UN AGENT THERAPEUTIQUE

(30) Priority: 07.09.2005 US 221647
(43) Date of publication of application: 25.06.2008
(73) Proprietor: Boston Scientific Limited, St. Michael (BB)
(72) Inventor: NAIMARK, Wendy, Boston, MA 02118 (US); SHANK, Peter, Boylston, MA 01505 (US); PALASIS, Maria, Wellesly, MA 02481 (US); FREYMAN, Toby, Waltham, MA 02453 (US); PANOS, Anastasia, Athens 27 T.K. 162 33 (GR); EPSTEIN, Samuel, J., Watertown, MA 02472 (US); ROUSSEAU, Alexandra, Cambridge, MA 02139 (US)
(74) Representative: Peterreins, Frank
(86) International application number: PCT/US2006/034707
(87) International publication number: WO 2007/030534

(56) References cited:
- EP-A- 1 466 634
- WO-A-94/13268
- US-A1- 2003 120 339
- US-A1- 2004 254 635
- US-A1- 2005 060 020

## Description

### FIELD OF THE INVENTION

This invention relates generally to medical devices, such as stents, for delivering a therapeutic agent to body tissue of a patient, such as a body lumen. More particularly, the invention is directed to a stent comprising at least one pocket for containing a therapeutic agent as well as ways for making such stents. The invention is also directed to a method for delivering therapeutic agents to body tissue of a patient.

### BACKGROUND OF THE INVENTION

A variety of medical conditions have been treated by introducing an insertable medical device having a coating for release of a therapeutic agent. For example, various types of medical devices coated with a therapeutic agent, such as stents, have been proposed for localized delivery of such agents to a body lumen. See, *e.g*., United States Patent No. 6,099,562 to Ding et al. issued on August 8, 2000. However, it has been noted that therapeutic agent delivery by means of medical devices can be improved.

In particular, the effectiveness of coated medical devices is limited by the surface area of the medical device. This problem is exacerbated when the medical device is used to delivery biopharmaceuticals, such as gene therapies and proteins. Generally, biopharmaceuticals have large therapeutic application windows. The use of coated medical devices makes the upper areas of these windows difficult or impossible to explore and test because of the limited carrying capacity of a coated medical device. The present invention provides a medical device that has increased carrying capacity to address this and other needs.

In US 2005/0060020 there is described a composite device comprising a first polymeric liner, a second polymeric liner, a structural member disposed between the liners and a pocket defined by the liners.

In US 2004/0254635 there is described an expandable device having a plurality of struts where at least one of the struts includes at least one opening into which a beneficial agent is loaded.

In EP 1 466 634 there is described a stent including a stent framework with plurality of reservoirs formed therein and a drug polymer positioned in the reservoirs.

In US 2003/0120339 there is described a stenting system including a tubular stent member, an outer membrane, an inner membrane, and a chamber located between the inner and outer membranes.

In WO 94/13268 there is described a device for treating and enlarging body lumens having delivery matrices comprising porous and erodible filaments.

### SUMMARY OF THE INVENTION

The present invention is defined by the features of the appended claims and seeks to address these needs by providing a stent having struts with pockets between at least one strut having at least one therapeutic agent.

In one embodiment, a medical device is provided for delivering a therapeutic agent comprising: (a) a stent having a sidewall comprising a plurality of struts, at least a first opening in the sidewall, and a first sidewall surface at least partially defined by the plurality of struts and the first opening; (b) a first layer disposed over at least a part of the first sidewall surface, wherein at least a portion of the first layer extends over a part of the first opening; (c) a second layer disposed over at least a part of the first sidewall surface, wherein at least a portion of the second layer is disposed over the portion of the first layer that extends over the first opening, (d) at least a first pocket disposed about at least a portion of the first opening; wherein the pocket is defined at least in part by the first layer and by the second layer; and (e) a therapeutic agent contained in the first pocket.

In another example not claimed by the invention, a medical device is provided for delivering a therapeutic agent comprising: (a) a stent having a sidewall comprising a plurality of struts, at least a first opening in the sidewall, an outer sidewall surface at least partially defined by the plurality of struts and the first opening, and an inner sidewall surface at least partially defined by the plurality of struts and the first opening; (b) a first layer disposed over at least a portion of the outer sidewall surface, wherein at least a portion of the first layer extends over a part of the first opening, and wherein the first layer is bound to at least a portion of the stent; (c) a second layer disposed over at least a portion of the inner sidewall surface, wherein at least a portion of the second layer extends over the first opening, (d) at least a first pocket disposed about at least a portion of the opening; wherein the pocket is defined at least in part by the first layer and at least in part by the second layer; and (e) a therapeutic agent contained in the first pocket.

In another example not claimed by the invention, a medical device is provided for delivering a therapeutic agent comprising: (a) a stent having a sidewall comprising a plurality of struts, at least a first opening in the sidewall, an outer sidewall surface defined by the plurality of struts and the opening, and an inner sidewall surface defined at least partially by the plurality of struts and the opening; (b) a first layer disposed over at least a portion of the outer sidewall surface, wherein at least a portion of the first layer extends over a part of the first opening; (c) a second layer disposed over at least a part of the inner sidewall surface, wherein at least a portion of the second layer extends over the opening, and wherein the second layer is bound to at least a portion of the first layer, (d) at least a first pocket disposed about at least a portion of the opening; wherein the pocket is defined at least In part by the first layer and at least in part by the second layer; and (e) a therapeutic agent contained in the first pocket.

In yet another embodiment, A medical device for delivering a therapeutic agent comprising: (a) a stent comprising a sidewall comprising at least a first strut and a second strut, and at least a first opening in the sidewall, wherein the first strut and the second strut each comprise an outer surface, an inner surface and at least one side surface; (b) a first layer bound to the side surface of at least one of the first and second struts, wherein at least a portion of the first layer extends over a portion of the first opening; (c) a second layer bound to the side surface of at least one of the first and second struts, wherein at least a portion of the second layer extends over a portion of the first opening; (d) at least a first pocket disposed about at least a portion of the opening; wherein the pocket is defined at least in part by the first layer and at least in part by the second layer; and (e) a therapeutic agent contained in the first pocket. The first layer may be bound to the stent.

The second layer may be bound to the stent. The second layer may be disposed over at least part of the first layer that is disposed over the first sidewall surface.

The first pocket may be co-extensive with the opening. The first pocket may be within the first opening. The first pocket may extend beyond the first opening. The medical device may further comprise a second pocket disposed about the opening.

At least one strut may comprise a side surface. The first and/or second layer may be disposed over the side surface.

The sidewall may further comprise a second sidewall surface. The first and/or second layer may be disposed over the second sidewall surface.

The stent further may comprise a second opening. A second pocket may be disposed about the second opening. The second pocket may contain a therapeutic material. The second pocket may contain a different therapeutic material than the first pocket The second pocket may be disposed about the first opening. The first and second pockets may be interconnected.

At least one of the first and second layers may comprise a plurality of sub-layers. At least two sub-layers may be comprised of a different material. At least two sub-layers may be of different thicknesses.

The medical device may further comprise a barrier between the first and second layers. The medical device may further comprise a third layer.

The first and second layers may be comprised of the same material, or different materials. The first and second layers may have different tensile strengths. The first and second layers may be of different thicknesses.

At least one of the first and second layer may be capable of being ruptured by the expansion of the stent. At least a portion of at least one of the first and second layer may comprise a plurality of pores. At least one of the first layer and second layer may comprise at least one preformed imprint. The imprinted area may generally have a lower tensile strength than the remainder of the layer.

At least one of the first layer and second layer may comprise a self-sealing material. At least one of the first layer and second layer may comprise a biodegradable material. At least one of the first layer and second layer may be substantially flexible.

The therapeutic agent may be releasable from the first pocket through at least one of the first layer and second layer. The therapeutic agent may be releasable from the first pocket after the expansion of the stent.

A method for making a medical device is also provided comprising the steps of: (a) providing a stent comprising a sidewall having an inner surface, an outer surface, at least one opening in the sidewall; wherein the sidewall comprises a plurality of struts, wherein the struts have an outer surface, an inner surface, and at least one side surface; (b) applying a first layer to a surface of the sidewall, and bonding at least a portion of the first layer to a surface of at least one strut, and covering at least one opening; (c) applying a second layer to a surface of the sidewall and bonding at least a portion of the second layer to a surface of at least one strut, and covering at least one opening, forming at least one pocket is generally disposed in at least one opening.

Another method not claimed by the invention for making a medical device comprising the steps of: (a) providing a prefabricated stent having an inner surface, an outer surface, and a sidewall comprising a plurality of struts having a plurality of openings therein; (b) applying a first layer disposed on the inner surface to form a covering over least a portion of the inner surface and at least one of the openings therein, so that at least a portion of the first layer is bonded to at least a portion of the inner surface; (c) applying a second layer disposed on the outer surface, so that at least a portion of the second layer is bonded to at least a portion of the outer surface, and so that at least one opening is located between the first layer and the second layer to form at least one pocket between the struts.

Another method of making a medical device is described comprising the steps of: (a) providing a stent comprising a sidewall having a first surface, a second surface, at least one opening in the sidewall; wherein the sidewall comprises a plurality of struts, wherein the struts have at least one surface; (b) applying a first layer about the first surface of the sidewall, and covering at least one opening; (c) applying a second layer to at least a portion of the first layer, and covering at least one opening, forming at least one pocket is generally disposed about at least one opening.

Another method not claimed by the invention of making a medical device is described comprising the steps of: (a) providing a stent comprising a sidewall having a first surface, a second surface, at least one opening in the sidewall; wherein the sidewall comprises a plurality of struts, wherein the struts have at least one surface; (b) applying a first layer to the first surface of the sidewall, and bonding at least a portion of the first layer to a surface of at least one strut, and covering at least one opening; (c) applying a second layer to the second surface of the sidewall, bonding at least a portion of the second layer to the surface of at least one strut, and covering at least one opening, forming at least one pocket is generally disposed about at least one opening.

Another method of making a medical device is described comprising the steps of: (a) providing a stent comprising a sidewall having a first surface, a second surface, at least one opening in the sidewall; wherein the sidewall comprises a plurality of struts, wherein the struts have at least one surface; (b) applying a first and second layers about the first surface of the sidewall, covering at least one opening, and forming at least one pocket is generally disposed about at least one opening.

Another method of making a medical device is described comprising the steps of: (a) providing a stent comprising a sidewall having a first surface, a second surface, at least one opening in the sidewall; wherein the sidewall comprises a plurality of struts, wherein the struts have at least one side surface; (b) applying a first and second layers about at least one side surface of at least one strut, covering at least one opening, and forming at least one pocket is generally disposed about at least one opening.

The method may further comprise the step of applying at least one therapeutic agent to at least a portion of the stent. The method may further comprise the step of inserting at least one therapeutic agent into at least one pocket.

The method may further comprise forming at least one imprint on the first and/or second layer. At least one imprint may be formed using a mandrel.

The method may further comprise the step of coating at least one of the first layer and second layer with a therapeutic agent.

### BRIEF DESCRIPTION OF THE DRAWINGS

Preferred features of the present invention are disclosed in the accompanying drawings, wherein similar reference characters denote similar elements throughout the several views, and wherein:

**FIG. 1A** is a side view of an exemplary stent suitable for the present invention;

**FIG. 1B** is a partial cross-sectional view of the stent of **FIG. 1A** along line **A-A;**

**FIG. 2A** **is** a partial side view of an exemplary stent where pockets are disposed within the openings;

**FIG. 2B** is a partial side view of an exemplary stent where pockets extend to the boundaries of the openings;

**FIG. 2C** is a partial side view of an exemplary stent where two or more pockets are disposed about an opening;

**FIG. 2D** is a partial side view of an exemplary stent where a pocket is disposed about more than one opening;

**FIGS 3A-3B** are partial cross-sectional views of an exemplary stent with pockets formed by layers disposed along the outer sidewall of the stent;

**FIGS. 3C-3D** are partial cross-sectional views of an exemplary stent with pockets formed by layers disposed along the inner sidewall of the stent;

**FIG. 4** is a partial cross-sectional view of an exemplary stent with pockets formed both by layers disposed along the outer sidewall of the stent and the inner sidewall of the stent;

**FIGS. 5-7** are a partial cross-sectional view of an exemplary stent with further embodiments of pockets formed by layers disposed along a sidewall of the stent;

**FIGS. 8A-8O** are enlarged partial cross-sectional views of pockets formed from two layers disposed in openings between adjacent struts; the examples shown in **FIGS. 8J, 8L****,** and **8M** are not claimed by the invention;

**FIGS. 9A-9C** are enlarged partial cross-sectional views of pockets formed from three layers disposed in openings between adjacent struts; the examples shown in **FIGS. 9B and 9C** are not claimed by the invention;

**FIGS. 10A-10F** are partial cross-sectional views of an exemplary stent with pockets formed by layers disposed on both the outer and inner sidewalls of the stent; the examples shown in **FIGS. 10A, 10B****,** **10C****,** **10F** are not claimed by the invention;

**FIGS. 11A-11R** are enlarged partial cross-sectional views of pockets formed from at least two layers disposed in openings between adjacent struts; the examples shown in **FIGS. 11A, 11B**, **11E,11F****,****11H,11I****,****11J,11K****,****11M,11N,11O****,****11P,11Q,11R** are not claimed by the invention

**FIG.12A** is an exemplary cross-sectional view of a stent with struts in a compressed state;

**FIG. 12B** shows the stent of **FIG. 12A** with a first layer;

**FIG. 12C** shows the stent of **FIG. 12B** with amounts of therapeutic material placed at or near the first layer;

**FIG. 12D** shows the stent of **FIG. 12C** with a second layer, forming pockets around the amounts of therapeutic material;

**FIG. 12E** shows the stent of **FIG. 12D** in an expanded state, the second layer having ruptures;

**FIG. 12F** shows the stent of **FIG. 12E** with the content of the pockets dispersing;

**FIGS. 13A-13F** is another embodiment of the method described in **FIGS. 12A-12F****;** and

**FIGS.14A-14D** are enlarged partial cross-sectional views of pockets formed from two layers disposed in openings between adjacent struts, wherein the adjacent struts are of varying shapes and sizes. The examples shown in **FIGS. 14A, 14B**, and 14D are not claimed by the invention.

### DETAILED DESCRIPTION OF THE INVENTION

### A. Suitable stents

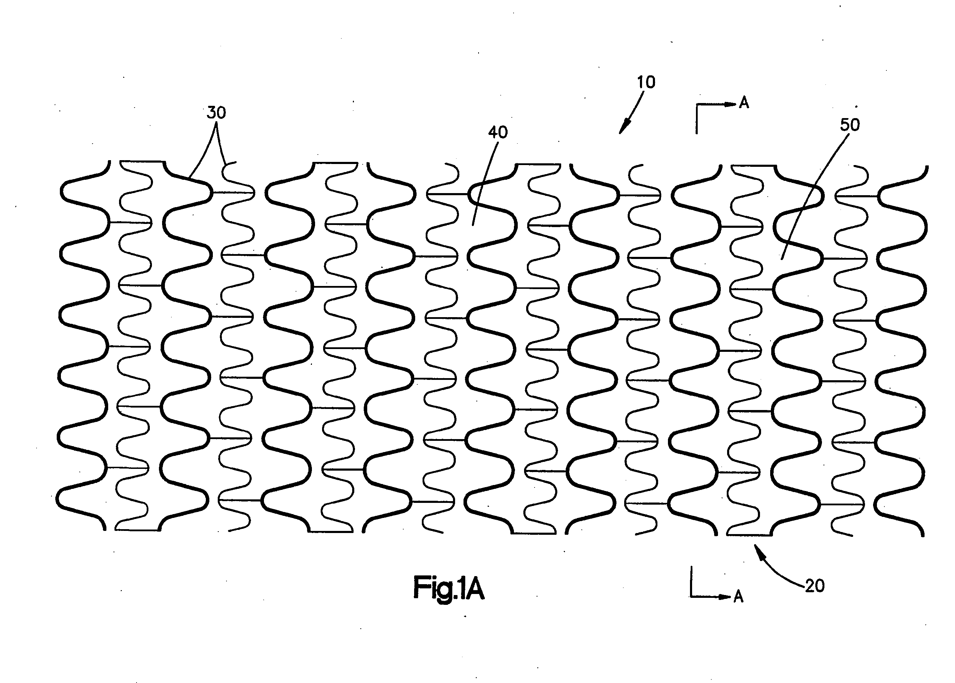

The invention described in detail herein generally relates to a stent having at least one opening in which at least one pocket is disposed about the opening. Suitable stents include ones that are used for cardiovascular, urinary and other medical applications. **FIG.1A** shows an example of a stent suitable for the present invention. In this example, the stent **10** comprises a sidewall **20** which comprises a plurality of struts **30** and at least one opening **40** in the sidewall **20.** Generally, the opening **40 is** disposed between adjacent struts **30.** Also, the sidewall **20** may have a first sidewall surface **50** and an opposing second sidewall surface **60,** which is not shown in **FIG. 1A****.** The first sidewall surface **50** can be an outer sidewall surface, which faces the body lumen wall when the stent is implanted, or an inner sidewall surface, which faces away from the body lumen wall. Likewise, the second sidewall surface **60** can be an outer sidewall surface or an inner sidewall surface. If the first sidewall surface is the outer sidewall surface, the second sidewall surface is the inner sidewall surface. If the first sidewall surface is the inner sidewall surface, the second sidewall surface is the outer sidewall surface.

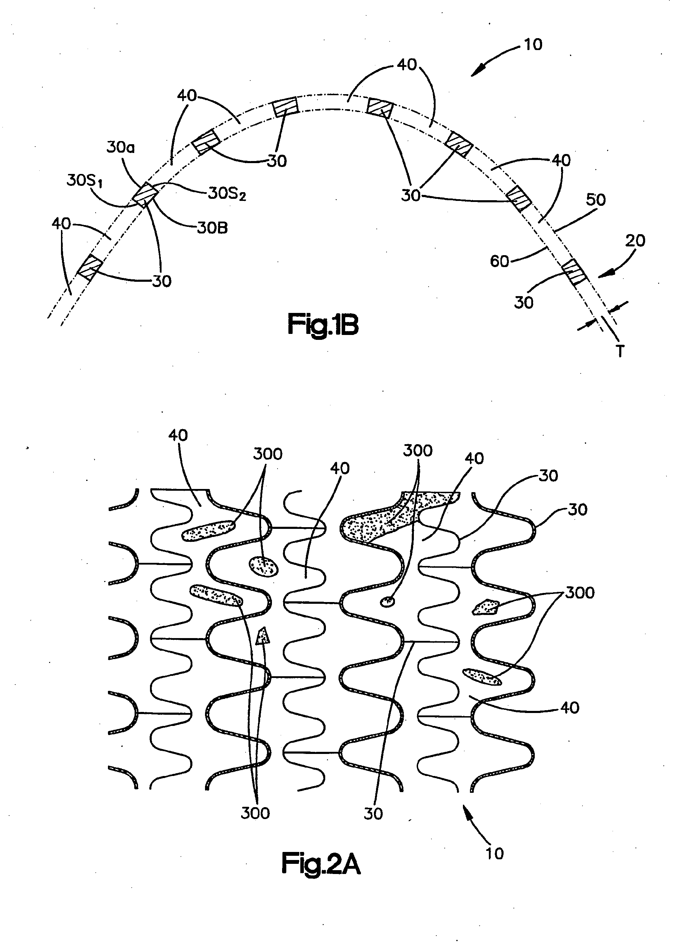

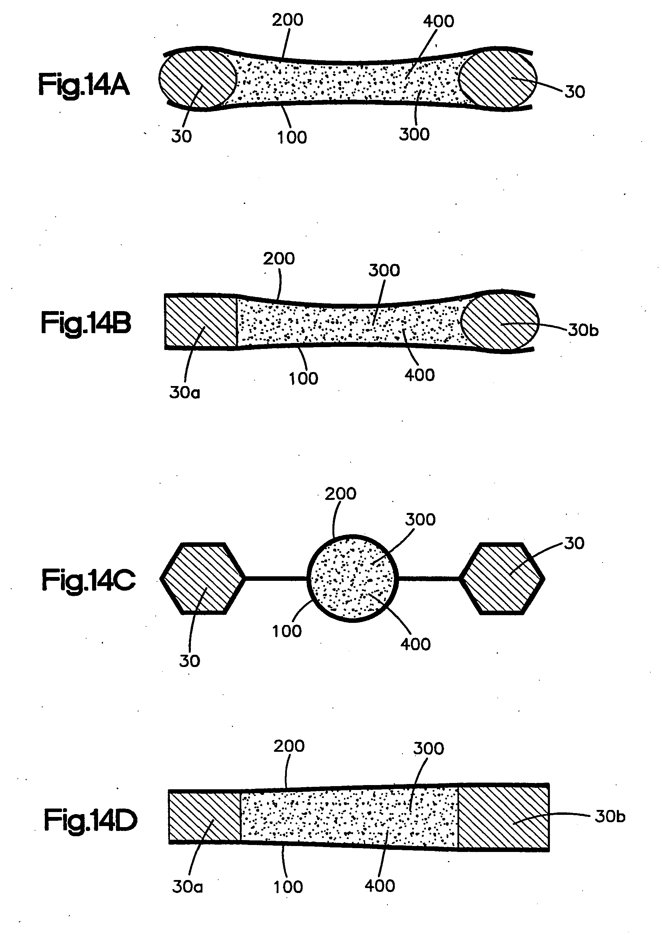

**FIG.1B** shows a cross-sectional view of the stent **10** in **FIG. 1A** along line **A-A.** The sidewall **20** may comprise a plurality of struts **30.** Each strut **30** may have an outer surface **30u,** which may generally be the surface of the strut **30** that faces the body lumen wall when the stent is implanted, and an inner surface **301,** which may generally be the surface facing away from the body lumen wall when the stent is implanted. Struts **30** may also have side surfaces **30s₁, 30s₂,** which may be disposed between the outer and inner strut surfaces **30u, 301.** The cross-sections of the struts **30** can be of any suitable shape (*see, infra,* **FIGS. 14A-14D****).**

As shown in **FIG.1A****,** the sidewall **20** has a thickness **T.** Furthermore, the sidewall may have a first sidewall surface **50,** which in this example is the outer surface of the sidewall. The first sidewall surface **50** may be defined by the openings **40** and the struts **30.** The sidewall may also have a second sidewall surface **60,** which in this example is the inner surface of the sidewall. The strut outer surface **30u** may generally lie along the outer sidewall surface **50.** The strut inner surface **301** may generally lie along the inner sidewall surface **60.**

Other suitable stents include, for example, intravascular stents such as those described in United States Patent No. 6,478,816 to Kveen et al.*,* for "Stent", issued on November 12, 2002. Suitable stents include self-expanding stents and balloon expandable stents. Examples of self-expanding stents useful in the present invention are illustrated in United States Patent Nos. 4,655,771 and 4,954,126 issued to Wallsten and 5,061,275 issued to Wallsten et al. Examples of appropriate balloon-expandable stents are shown in United States Patent No. 5,449,373 issued to Pinchasik et a/.

Stents that are suitable for the present invention may be fabricated from metallic, ceramic, or polymeric materials, or a combination thereof. Metallic materials are more preferable. Suitable metallic materials include metals and alloys based on titanium (such as nitinol, nickel titanium alloys, thermo-memory alloy materials), stainless steel, tantalum, nickel-chrome, or certain cobalt alloys including-cobalt-chromium-nickel alloys such as Elgiloy® and Phynox®. Metallic materials also include clad composite filaments, such as those disclosed in WO 94/16646.

Suitable ceramic materials include, but are not limited to, oxides, carbides, or nitrides of the transition elements such as titaniumoxides, hafnium oxides, iridiumoxides, chromium oxides, aluminum oxides, and zirconiumoxides. Silicon based materials, such as silica, may also be used.

The polymer(s) useful for forming the stent should be ones that are biocompatible and avoid irritation to body tissue. They can be either biostable or bioabsorbable. Suitable polymeric materials include without limitation polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for stents include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly(γ-caprolactone), poly(γ -hydroxybutyrate), polydioxanone, poly(γ -ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or crosslinking groups, *e.g*., RGD, in which the polymers retain their structural integrity while allowing for attachment of cells and molecules, such as proteins, nucleic acids, and the like.

### B. The Pockets

Pockets **300** may be disposed in openings **40.** As discussed in greater detail below, pockets **300** may be of various shapes and sizes. Pockets **300** may also be situated about openings **40** in a variety of manners. A single stent **10** may have several different types of pockets **300.** Numerous variations and applications will be appreciated by those skilled in the art.

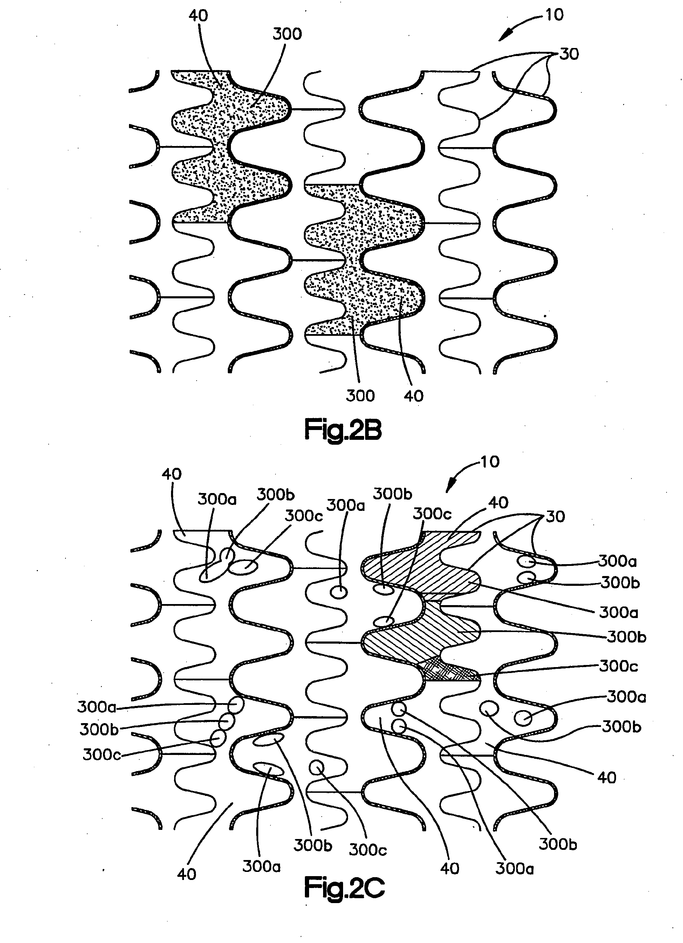

**FIG. 2A** shows exemplary pockets **300** disposed about the openings **40** in the present invention can be situated completely within the boundaries of the openings. These pockets **300** do not contact the boundaries of the openings **40,** which are generally defined by struts **30.** In other embodiments, such as those shown in **FIG. 2B****,** the pockets **300** can contact boundaries of the openings **40,** which are generally defined by the struts **30.** In some such embodiments, the pockets **300** may be coextensive with the opening **40.**

In yet other embodiments, such as those shown in **FIG. 2C****,** a plurality of pockets **300a, 300b, 300c** can be disposed about a single opening **40.** The number of pockets **300** that can be disposed about an opening **40** can vary from opening to opening within a stent **10**. For example, a first pocket **300a** can be disposed about a first opening **40** in a stent **10,** while, two or more pockets **300b, 300c** can be disposed about a second opening **40.** When two or more pockets **300** are disposed about an opening **40,** the pockets can have various shapes and sizes as shown in **FIG. 2C****.** Also, the two or more pockets 300 disposed about an opening **40** can be separated from each other or, some or all of the pockets can be in contact with each other.

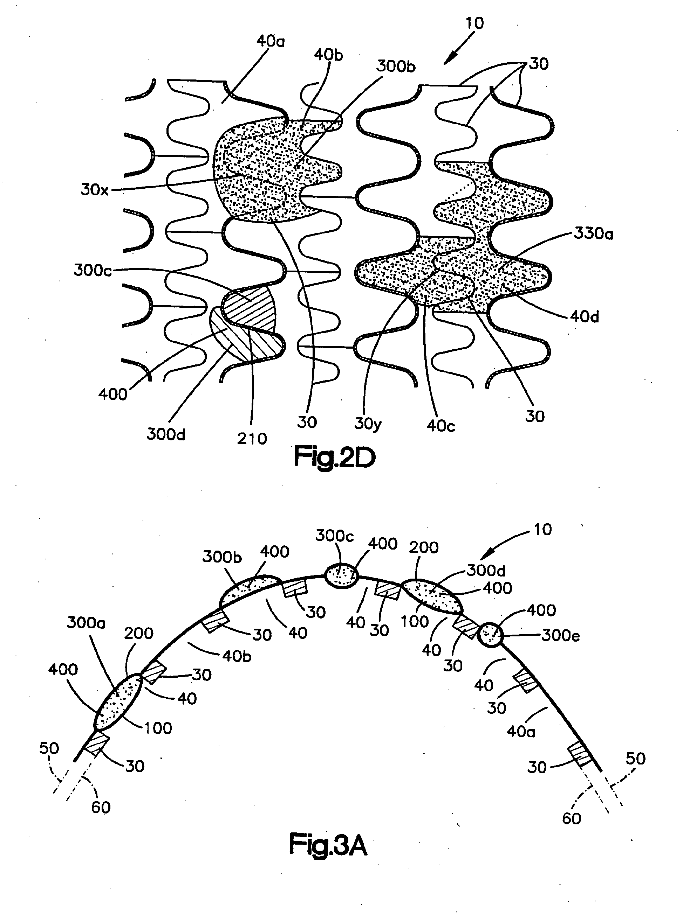

In other embodiments, a pocket **300** can be disposed about two or more openings **40a, 40b** as shown in **FIG. 2D****.** Pockets **300** can be coextensive with some or all of the openings **40,** as shown with pocket **300a** in **FIG. 2D****.** Alternatively, the pocket **300** can be disposed about only portions of the openings **40,** such as shown in pocket **300b.** Also, in some embodiments, the pocket **30** can be disposed over the outer sidewall surface, as shown in pocket **300b** so that strut **30x** is covered by the pocket **300b.** In other embodiments, the pocket **300a** can be disposed over the inner sidewall surface, so that strut **30y** lies above the pocket **300a.** In yet other embodiments, the pocket **300** can be disposed about a strut **30** so that a part of the pocket is disposed over the strut and part of the pocket is disposed under the pocket (discussed in more detail below).

As also shown in **FIG. 2D****,** combined amounts of therapeutic material **400** disposed within pockets **300c, 300d** may be in communication with each other by way of an orifice **210.** At least one therapeutic agent may be present in an amount of therapeutic material **400.** The orifice **210** may be open or closed during the implantation of the stent **10.** The amounts of therapeutic material comprising a combined amount of therapeutic material **400** are in at least two different openings **40.** Combined amounts of therapeutic material **400** may be beneficial to combine the contents of more than one amount of therapeutic material together upon release of the contents in the body. For instance, it may be beneficial for two amounts of therapeutic material **400a, 400b** comprising a combined amount of therapeutic material **400** to be initially provided with two separate substances, but desirable for those substances to remain at least partially separate until the substances are released from the individual amounts of therapeutic material. For example, first and second adjacent amounts of therapeutic material **400a, 400b** may comprise a combined amounts of therapeutic material **400,** wherein.the first adjacent amount of therapeutic material **400a** is comprised with a inactive cells and the second adjacent pocket is filled with active genes. Upon expansion of the stent **10** (discussed in detail below), the orifice **210** may rupture allowing the contents of the first and second adjacent amounts of therapeutic material **400a, 400b** combine before the combined amount of therapeutic material **400** itself ruptures and releases its combined contents into the body.

### C. Pockets With Layers Disposed Over The Same Sidewall Surface

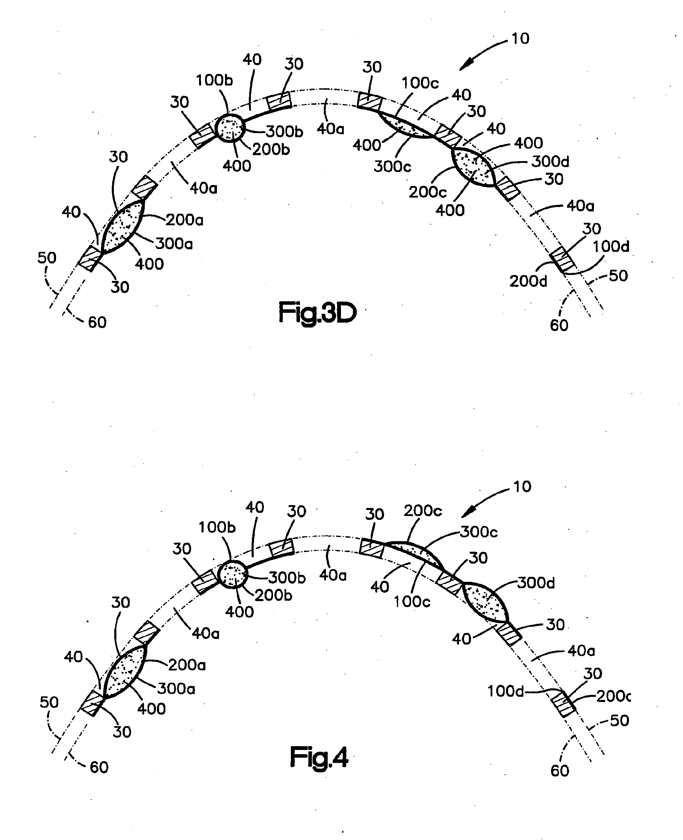

In one embodiment of the invention, a first layer **100** is disposed over at least a part of a first sidewall surface, which can be the outer or inner sidewall surface **50, 60.** At least a portion of the first layer **100** may extend over a part of an opening **40.** Moreover, a second layer **200** may also be disposed over at least a part of the first sidewall surface. At least a portion of the second layer **200** may be disposed over the portion of the first layer **100** that extends over the opening **40.** The first layer **100** may define a first surface of a pocket **300** and the second layer may define a second surface of the pocket **300.** The pocket **300** may be disposed about at least a portion of the opening **40.** A therapeutic agent **400** can be contained in the pocket **300.** **FIGS. 3A-3D** shows an example of such an embodiment where the layers defining the pocket are disposed over the same stent sidewall surface.

**FIG. 3A** shows a cross-section of a stent **10** with pockets **300.** In this example the first layer **100** and second layer **200** define a number of pockets **300a, 300b, 300c, 300d** and **300e.** The pockets may contain a therapeutic agent **400.** In this embodiment, both the first layer **100** and the second layer **200** are disposed over the same sidewall surface, which in this example is the outer sidewall surface **50.** Even though the first and second layers **100, 200** are disposed over the outer sidewall surface **50,** the pockets defined by the first and second layers **100, 200** can extend into the openings **40,** such as pockets **300a, 300c, 300d** and **300e.**

As discussed above, the pockets **300** can contact the boundaries of the openings **40** about which they are disposed. Pockets **300a** and **300d** in **FIG. 3A** are examples of such pockets. Pockets **300c** is an example of a pocket that is disposed within a single opening **40** and does not contact the boundaries of the opening **40.** Although the pockets **300** may be disposed within an opening **40,** the pocket can extend above or below the opening, such as the tops of the pockets **300,** which may be defined by the first layer **100.** In addition, pockets **300** can be disposed about an opening **40** when the pocket is disposed over or under an opening such as pocket **300b.** Also, in a stent with pockets **300,** some openings **40** can be free of pockets, such as opening **40a.** Furthermore, although the first and second layers **100, 200** can extend over an opening **40,** the layers **100, 200** do not have to form a pocket, such as in opening **40b.**

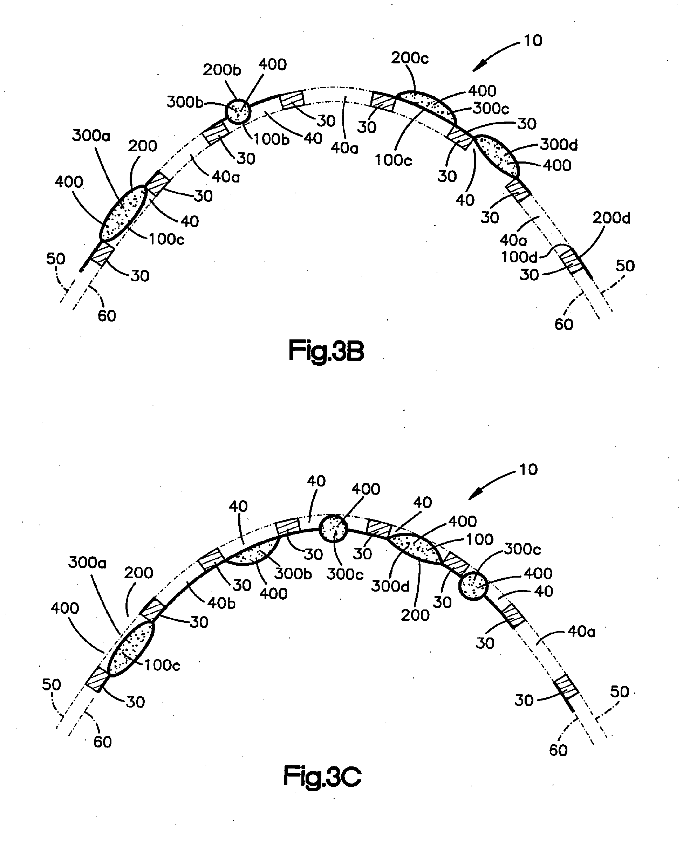

**FIG. 3B** shows embodiments of pockets **300** similar to those shown in **FIG. 3A****,** but with some openings **40a** free of pockets spaced between pockets **300a-300e.** The result of this arrangement is that separate sets of first **100a, 100b, 100c** and second layers **200a, 200b, 200c** may define one or more pockets **300.** In this embodiment, first and second layers **100a, 200a** define pocket **300a,** first and second layers **100b, 200b,** which can be referred to as third and fourth layers, define pocket **300b,** and first and second layers **100c, 200c,** which can be referred to as fifth and sixth layers, define pockets **300c, 300d.**

**FIG. 3C** shows a cross-section of a stent **10** with pockets **300** similar to those shown in **FIG. 3A****.** However, in this embodiment, the first and second layers **100, 200** are disposed over the inner sidewall surface **60.** **FIG**. **3D** again shows embodiments of pockets **300** similar to those shown in **FIG. 3C****,** but with more openings **40a** free of pockets spaced between pockets **300a-300d.** As previously discussed, first and second layers **100, 200** may form numerous pockets **300.**

**FIG. 4** shows another embodiment of a stent **10** with pockets **300.** In this embodiment, different sets of first and second layers **100, 200** are disposed over the inner sidewall surface **60** at one or more locations to form pockets **300a, 300b,** and also disposed over the outer sidewall surface **50** at one or more locations to form pockets **300c, 300d.** In this embodiment, first and second layers **100a, 100b** form pocket **300a,** which is coextensive over an opening **40** on inner sidewall surface **60.** Layers **100b, 200b** form a bulbous pocket **300b,** which is off-center in opening **40** on inner sidewall surface **60.** Layers **100c, 200c** collectively form two pockets **300c, 300d** disposed along outer sidewall surface **50.**

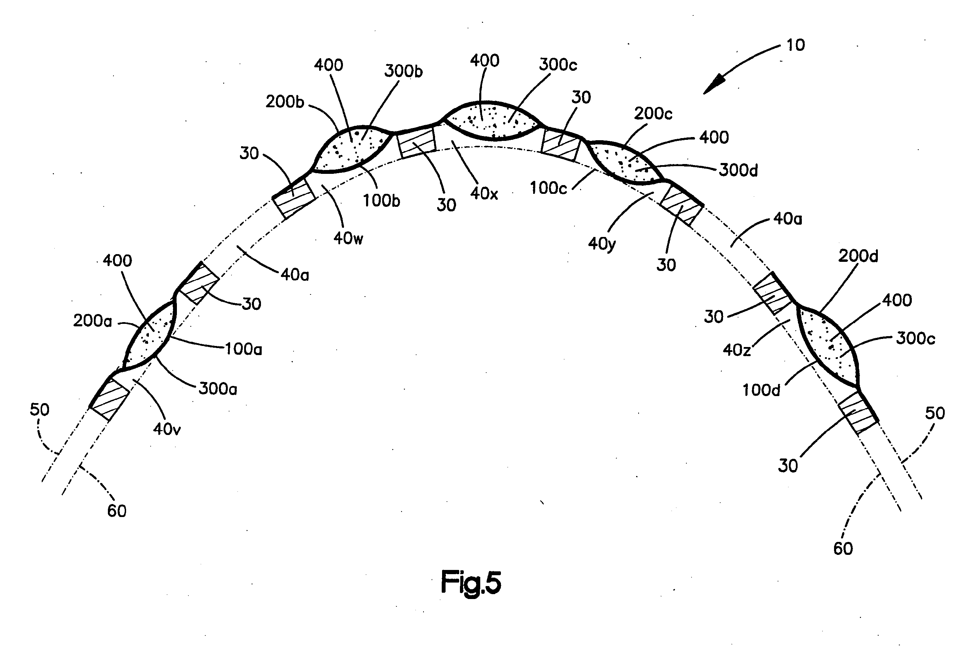

**FIG. 5** shows another embodiment of the inventions. In this embodiment, neither the first and second layers **100, 200** are entirely disposed over a common opening **40.** For instance, first layer **100a** is disposed over opening **40v** and the two adjacent struts **30.** However, second layer **200a** is disposed over first layer **100a,** but not over the two adjacent struts **30** that are adjacent opening **40v.** Likewise, pocket **300b** is defined by second layer **200b,** which is disposed over two adjacent struts **30** and first layer **100b,** which is not disposed over the two adjacent struts **30** adjacent opening **40w.** Similarly, pockets **300c** and **300d** are formed by a first or second layer that is disposed over just one strut that is adjacent the respective opening over which each pocket is disposed. Lastly, pocket **300e** is defined by a first layer **100d,** which is disposed over one strut **30** adjacent opening **40z,** and a second layer **200d,** which is disposed over two adjacent struts **30** adjacent opening **40z** over which pocket **300e** is disposed.

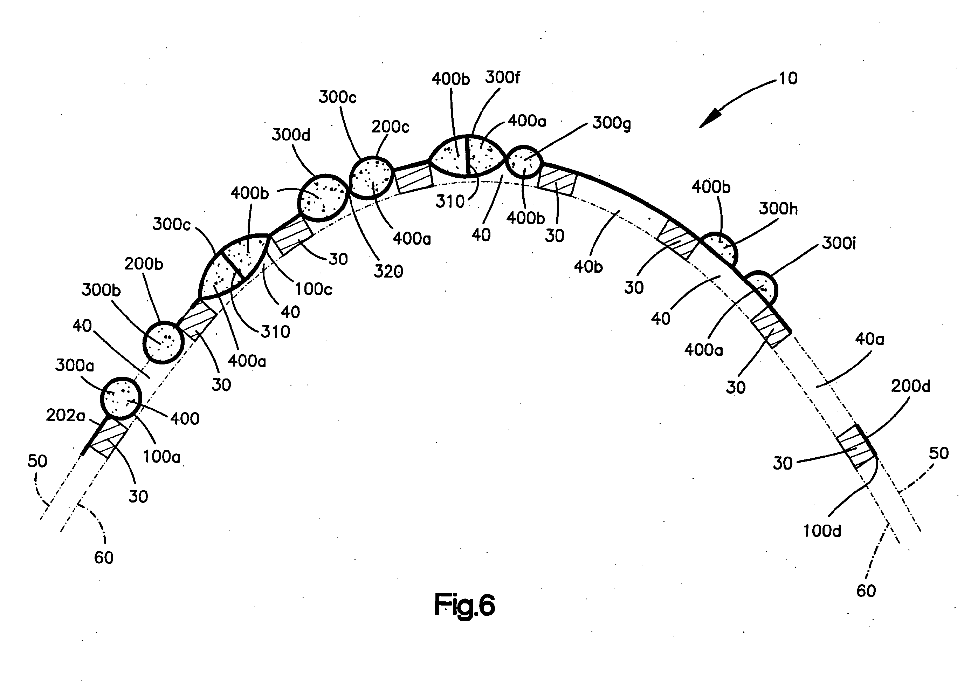

More than one pocket **300** may be situated about a single opening **40.** As seen in **FIG. 6****,** pockets **300a, 300b** are both situated about opening **40.** Moreover, pockets **300a, 300b** may not be in contact with each other, thereby each being formed by separate first **100a, 100b** and second layers **200a, 200b.**

**FIG. 6** also shows an embodiment of a stent **10** with pockets **300** wherein at least one pocket has more than one therapeutic material **400a, 400b** contained within it. An exemplary pocket **300** with more than one therapeutic material is seen in pocket **300c.** Therapeutic materials **400a, 400b** may be separated by a barrier **310** situated within a pocket **300.** Barrier **310** may be rupturable.

**FIG. 6** further shows embodiments of therapeutic materials **400a, 400b** contained in pairs of separate pockets **300d, 300e** and **300h, 300i.** Each pair of pockets may be situated about a single opening **40.** Combinations of the above-described pockets are also contemplated for a single opening, as seen with **pockets 300f and 300g.**

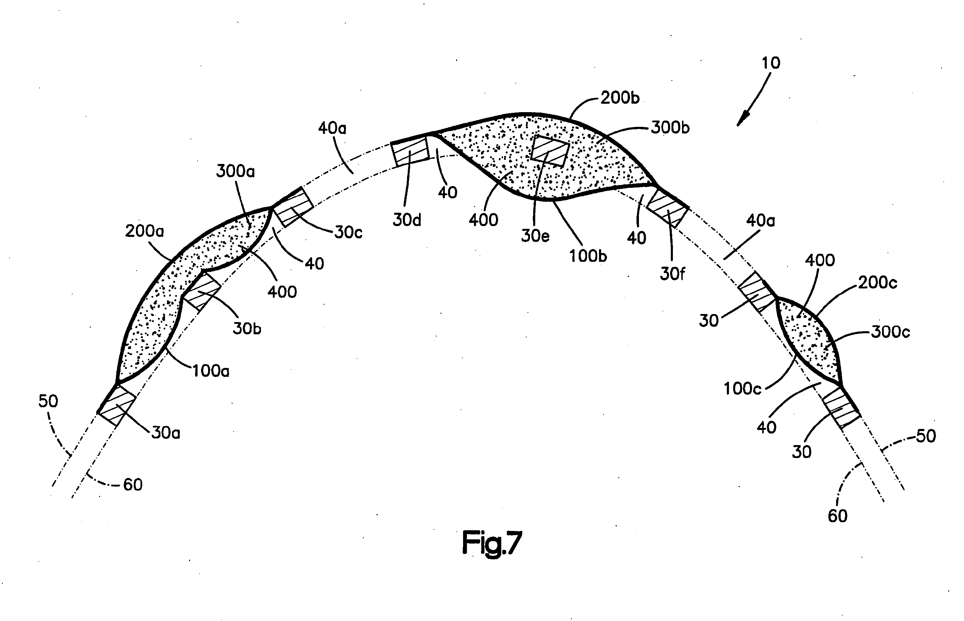

**FIG. 7** shows another embodiment of a stent **10** with pockets **300.** In this embodiment, pockets **300a, 300b** are situated about more than one opening **40.** Pocket **300a** is formed by first layer **100a,** which is in contact with three consecutive struts **30a, 30b, 30c** and second layer **200a,** which contacts first layer **100a** only at or near struts **30a** and **30c.** Pocket **300b** is formed in a similar manner across struts **30d, 30e, 30f,** except that in this embodiment, intermediate strut **30e** is enclosed within pocket **300b,** as first layer **100b** passes below strut **30e,** and second layer **200b** passes above strut **30e.** Pockets **300a, 300b** may therefore be larger than a pocket **300** formed solely in a single opening **40.**

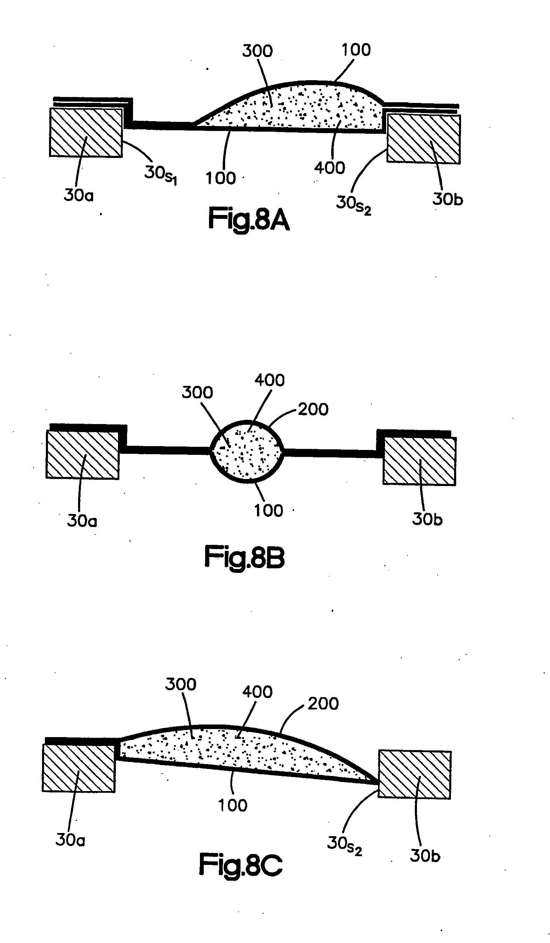

FIGS. **8A-8O** are enlarged partial cross-sectional views of pockets **300** formed from first and second layers **100, 200** disposed in openings **40** between adjacent struts **30a, 30b.** It is expressly contemplated that a pocket **300** could exhibit some or all of the characteristics of pockets described herein, and in detail below.

FIG. **8A** shows a pocket **30** formed by first and second **layers 100, 200** partially conforming to side surfaces **30s₁** and **30s₂** of adjacent struts **30a, 30b,** wherein the second layer **200** has a rise and first layer **100** is generally flat.

**FIG. 8B** shows a pocket **300** formed by first and second layers **100, 200,** wherein the pocket is generally formed by a circular bulge spaced apart from adjacent struts **30a, 30b.**

**FIG. 8C** shows a pocket **300** formed by first and second layers **100, 200,** wherein a boundary of the pocket is formed by layers **100, 200** terminating at side surface **30s₂** of strut **30b.**

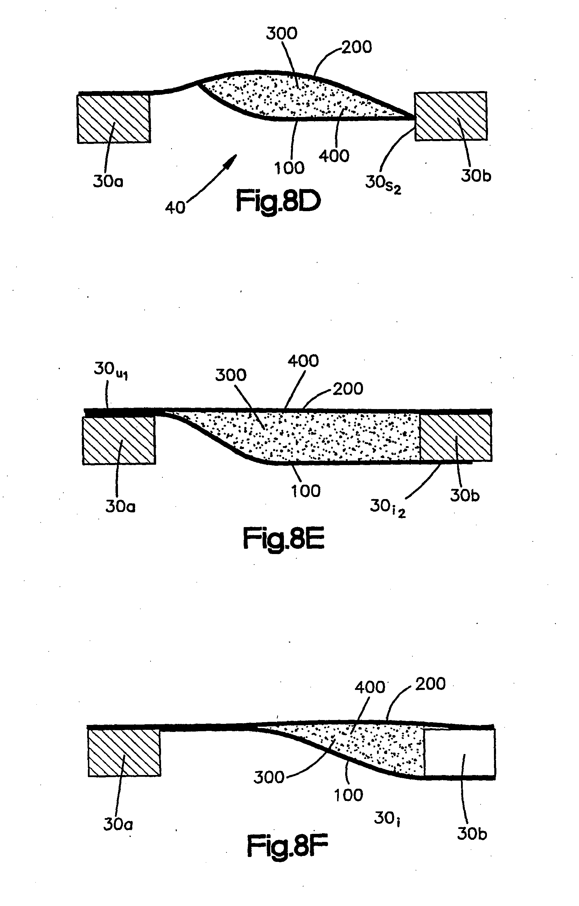

**FIG. 8D** shows a pocket **300** formed by first and second layers **100, 200,** wherein first and second layers **100, 200** terminate at side surface **30s₂** of strut **30b,** and first layer **100** does not extend across the entire opening **40** between adjacent struts **30a, 30b.**

**FIG. 8E** shows a pocket **300** formed by first and second layers **100, 200,** wherein first and second layers **100, 200** are situated on the outer surface **30u₁** of adjacent strut **30a,** but the first layer **100** is in contact with the inner surface **30i₂** of adjacent strut **30b,** while second layer **200** is in contact with the outer surface **30u₂** of adjacent strut **30b.** The arrangement seen about strut **30b** is discussed in more detail, *infra.* **FIG. 8F** shows a pocket **300** formed by a combination of the embodiments shown in **FIGS. 8D** and **8E****.**

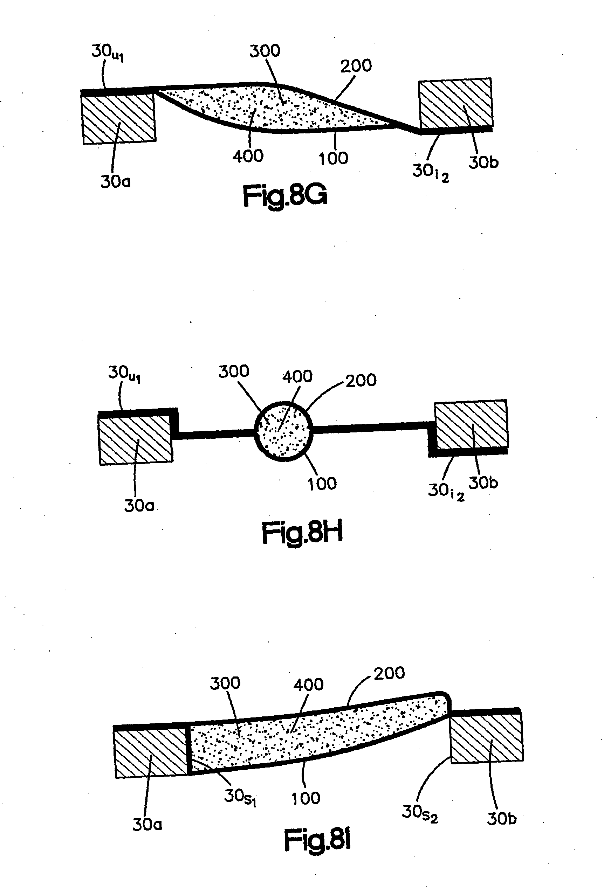

**FIG. 8G** shows a pocket **300** formed by first and second layers **100, 200** that are situated at or near outer surface **30u₁** of adjacent strut **30a,** and then at or near inner surface **30i₂** of adjacent strut **30b.** FIG. 8H shows a pocket formed by a combination of the embodiments shown in **FIGS. 8B** and **8G****.**

**FIG. 8I** shows a pocket **300** formed by first and second layers **100, 200,** wherein the first layer **100** is in contact with the entire side surface **30s₁** of adjacent strut 30a.

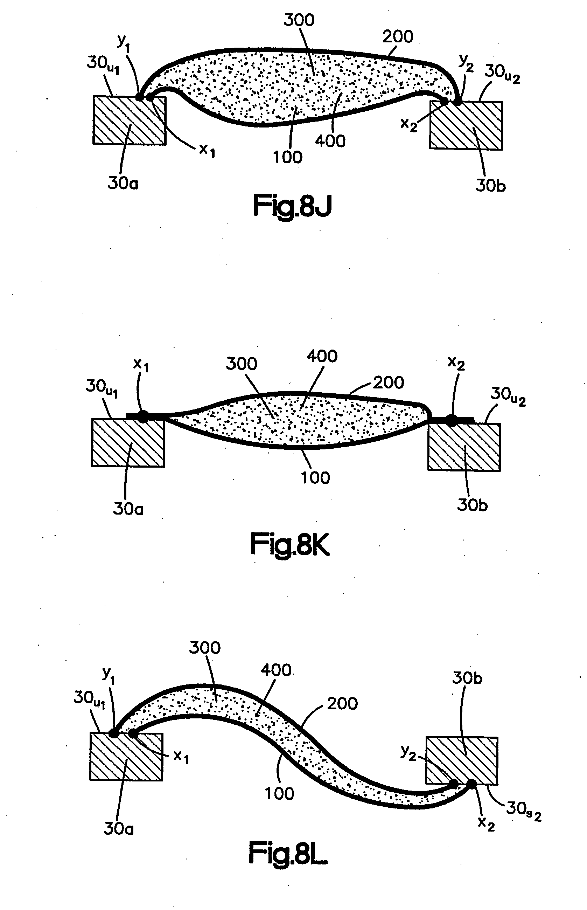

**FIGS. 8J** and **8L** show examples of pockets **300** formed by first and second layers **100, 200,** wherein the first and second layers are bonded to surfaces of adjacent struts **30a, 30b** and/or each other. Layers **100, 200** in **FIG**. **8J** are individually bonded to the outer surfaces **30u₁, 30u₂** of adjacent struts **30a, 30b,** respectively, First layer **100** may be bonded to outer surfaces **30u₁, 30u₂** at points **x₁, x₂**, respectively. Second layer **200** may be bonded to outer surfaces **30u₁, 30u₂** at points **y₁, y₂**, respectively. Layers **100, 200** in the embodiment shown in **FIG. 8K** are bonded to each other instead of adjacent struts **30a, 30b,** at points x₁ and x₂.

**FIG. 8L** combines the embodiment of **FIGS. 8G** and the example of **FIG 8J****.** Layers **100, 200** may be bonded to the outer surface **30u₁** of strut **30a** at points **x₁, y₁**, respectively, but then bonded to the inner surface **30i₂** of strut **30b** at points **x₂, y₂**, respectively. The resulting arrangement is a "wave" pattern.

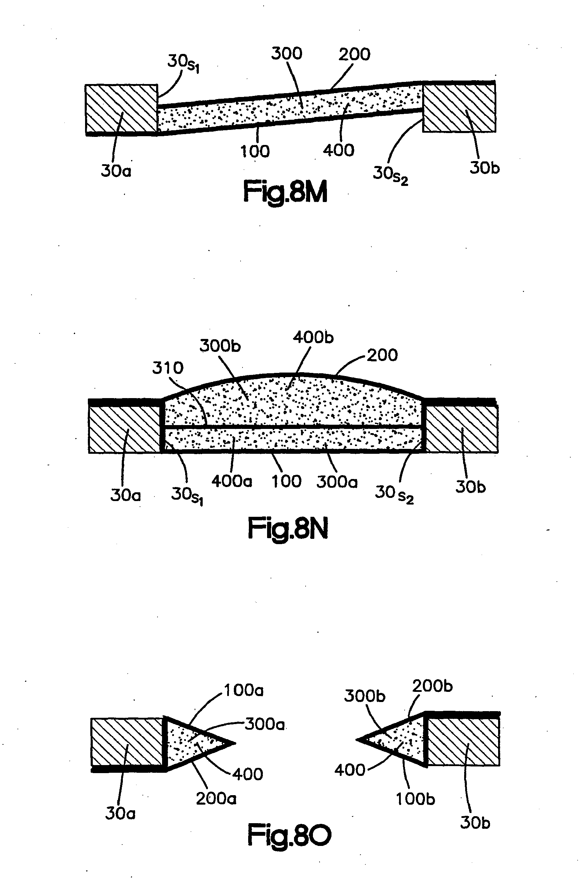

**FIG. 8M** shows a pocket **300** not claimed by the invention formed by first and second layers **100, 200** wherein the first layer **100** contacts the inner surface **30i₁** and is bonded to the side surface **30s₂** of strut **30b,** and the second layer **200** is bonded to the side surface **30s₁** of strut **30a** and contacts the outer surface **30u₂** of strut **30b.** The resultant arrangement is a "slanted" pattern.

**FIG. 8N** shows pockets **300a, 300b** formed by first and second layers **100, 200,** wherein a barrier **310** is utilized in a different manner than shown in **FIG. 6****.** Here, barrier **310** spans the opening, and contacts side surfaces **30s₁, 30s₂** of adjacent struts **30a, 30b,** resulting in a substantially horizontal arrangement barrier **310.** First pocket **300a** containing first therapeutic material **400a** is therefore separated from second pocket **300b** containing second therapeutic material **400b** by barrier **310.** Moreover, first layer **100** may contact the length of side surfaces **30s₁, 30s₂,** which in this case results in both pockets **300a, 300b** being at least partially formed by first layer **100.**

**FIG. 8O** shows pockets **300a, 300b** formed by first **100a, 100b** and second layers **200a, 200b,** wherein the pockets are separate from each other. **FIG. 8O** is a variation of the embodiments **300a, 300b** shown in **FIG. 6****.**

**FIGS**. **9A-9C** are enlarged partial cross-sectional views of pockets **300** formed from first, second, and third layers **100, 200, 500** disposed in openings **40** between adjacent struts **30a, 30b.** It is expressly contemplated that a pocket **300** could exhibit some or all of the characteristics of pockets described herein, and in detail below.

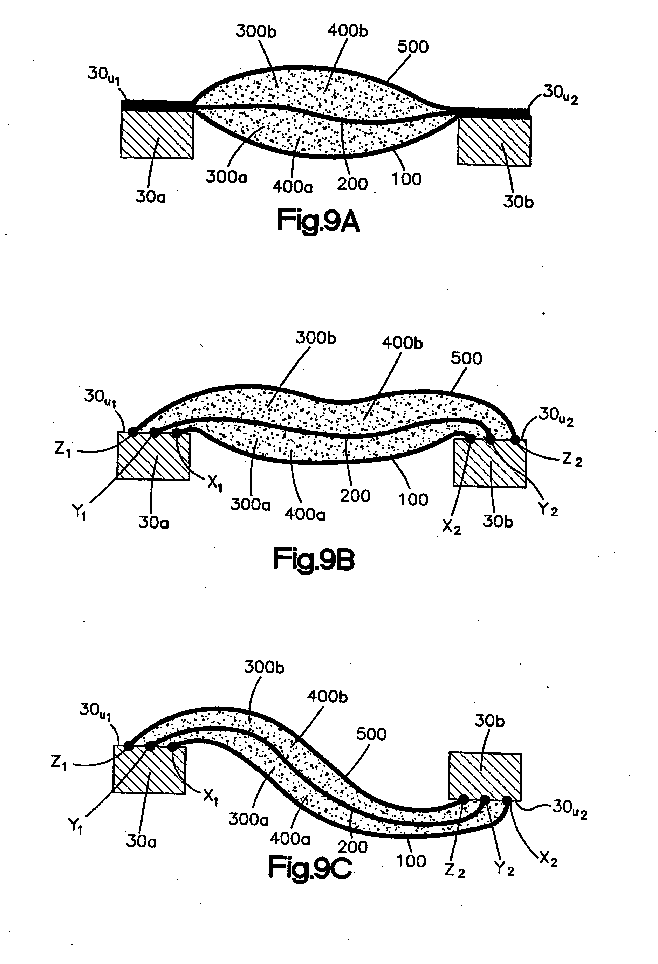

**FIG. 9A** shows pockets **300a, 300b** formed by layers **100, 200, 500,** wherein each layer is at or near the outer surfaces **30u₁, 30u₂** of adjacent struts **30a, 30b,** and each pocket is formed as a bulge between the struts. Pockets **300a, 300b** may have different therapeutic materials **400a, 400b,** within them, respectively. Second layer **200** may serve as a barrier in this embodiment.

**FIG. 9B** is similar to the example of **FIG. 8J****,** but includes a third layer **500** to form pocket 300b. Third layer may be bonded to outer surfaces **30u₁, 30u₂** of adjacent struts **30a,** 30b at points **z₁, z₂,** respectively. Pocket **300b** may contain a second therapeutic agent **400b.**

Similarly, **FIG. 9C** is similar to the example of **FIG. 8L****,** but includes a third layer **500** to form pocket **300b.** Third layer may be bonded to outer and inner surfaces **30u₁, 30i₂** of adjacent struts **30a, 30b** at points z₁, z₂, respectively. Pocket **300b** may contain a second therapeutic agent **400b.**

### D. Pockets with Layers Disposed Over Different Sidewall Surfaces

In another embodiment of the invention, a first layer **100** may be disposed over at least a portion of the inner sidewall surface **50,** and at least a portion of the first layer **100** may extend over a part of an opening **40.** Moreover, a second layer **200** may also be disposed over at least a part of the outer sidewall surface **60.** At least a portion of the second layer **200** may be disposed over the portion of an opening **40** that the first layer **100** that extends over. The first layer **100** may define a first surface of a pocket **300** and the second layer may define a second surface of the pocket **300.** The pocket **300** may be disposed about at least a portion of the opening **40.** A therapeutic agent **400** can be contained in the pocket **300.** At least one of the first and second layers **100, 200** may be bound to a sidewall surface **50, 60,** which may occur using heat, adhesive materials and/or chemicals, or other methods and materials known by those of skill in the art.

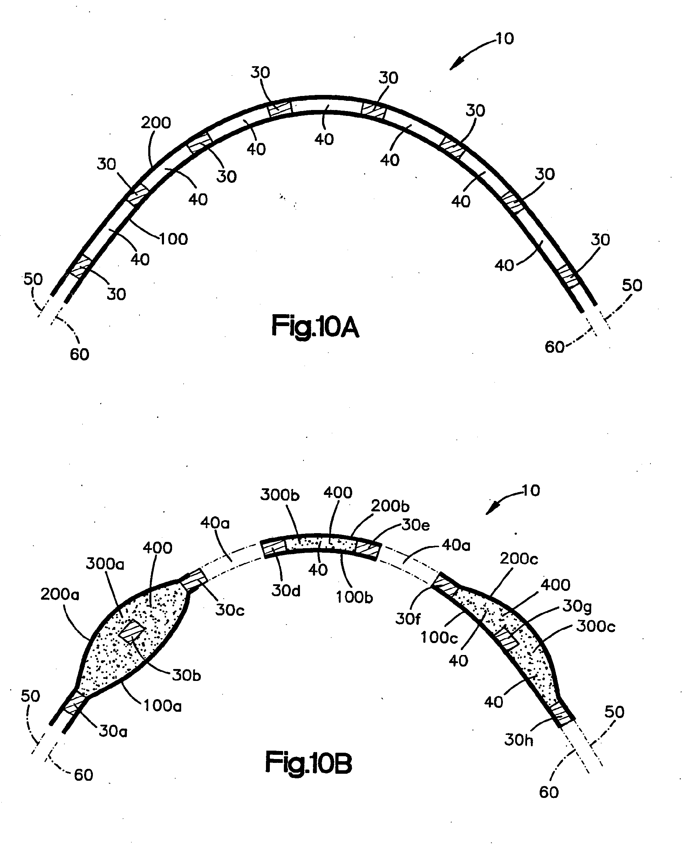

**FIG. 10A** shows a cross-section of a stent **10** with pockets **300** with first layer **100** disposed on the inner sidewall surface **60** and second layer **200** disposed on the outer sidewall surface **50.** The example not claimed by the invention of **FIG.10A****,** along with the related embodiments described below in relation to **FIGS. 10B-10F****,** may have any or all of the characteristics described, *supra,* in relation to the embodiments of **FIGS. 1A-9C****.**

**FIG. 10B** shows a stent **10** not claimed by the invention with several examples of pockets **300a, 300b, 300c** formed by different sets of first and second layers. Pocket **300a** is formed by layers **100a, 200a,** and encompasses strut **30b,** which is situated between struts **30a** and **30c.** This example is similar to pocket **300b** of **FIG. 7****.** Pocket **300c** is also related, except that first layer **100** is in contact with intermediate strut **30g.**

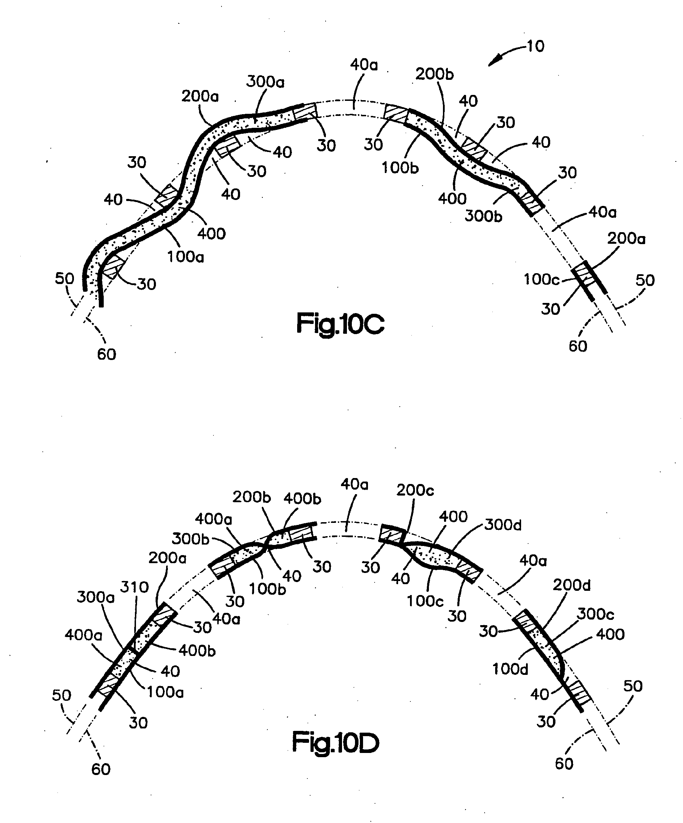

**FIG. 10C** shows further examples of pockets **300a, 300b** not claimed by the invention formed by different sets of first and second layers. Pocket **300a** results from a "weaved" arrangement of layers **100a, 200a,** wherein the first and second layers may contact only every other strut while providing a continuous pocket **300a** that is situated about more than one opening **40** and on the outer and inner sidewall surfaces **50, 60.** Pocket **300** is a related arrangement, except that the first layer **100b** sits at or beneath the inner sidewall surface **60.**

**FIG.10D** shows further embodiments of pockets **300a-300d** formed by different sets of first and second layers **100, 200,** which are variations of pockets described *supra,* but wherein the first **100** and second layers **200** are disposed over different sidewall surfaces. For example, pocket **300a** is formed by layers **100a, 200b,** with barrier **310** extending therebetween, and two therapeutic materials **400a, 400b** disposed therein. Pockets **300b, 300c** in this embodiment are formed in a single opening **40,** wherein a portion of layers **100b, 200b** are bound to each other. Pocket **300d** in this embodiment is formed by layers **100c, 200c,** wherein a portion of layer **200c** is disposed over an entire side surface of an adjacent strut **30.** Pocket **300e** in this embodiment is formed by layers **100e, 200e,** wherein layer **200e** does not extend over the entire opening **40.**

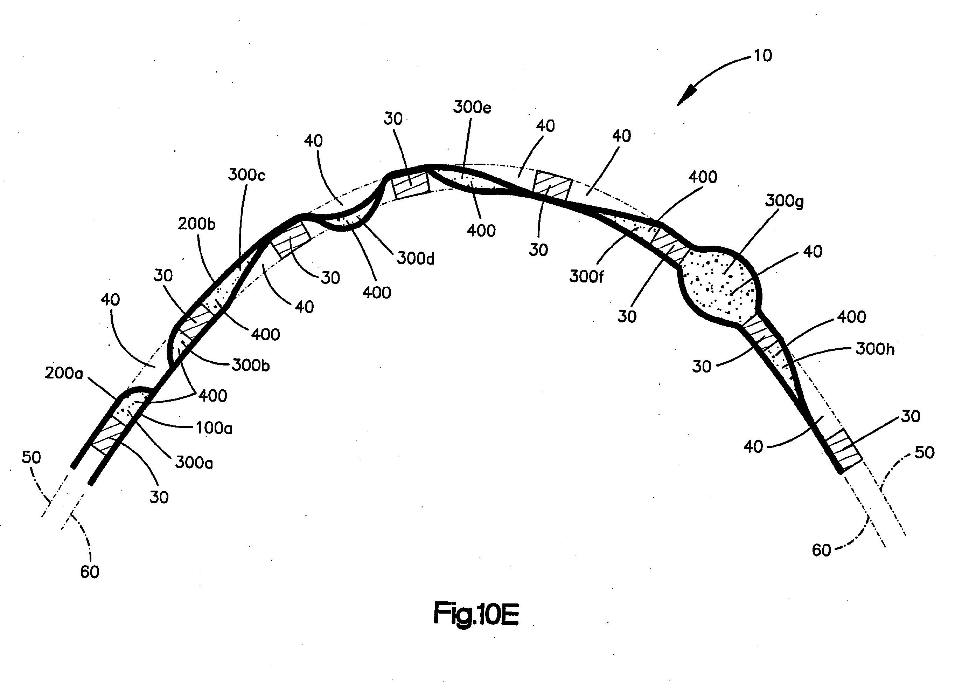

**FIG.10E** is an exemplary stent demonstrating the variations of pockets **300** that may be situated on a single stent **10,** using the teachings described above. As seen in the drawing, layers **100, 200** may or may not be continuous, and may or may not extend completely over openings. Moreover, layers **100, 200** may or may not be disposed over the same sidewall surface. Pockets **300** may vary in size and shape from opening to opening.

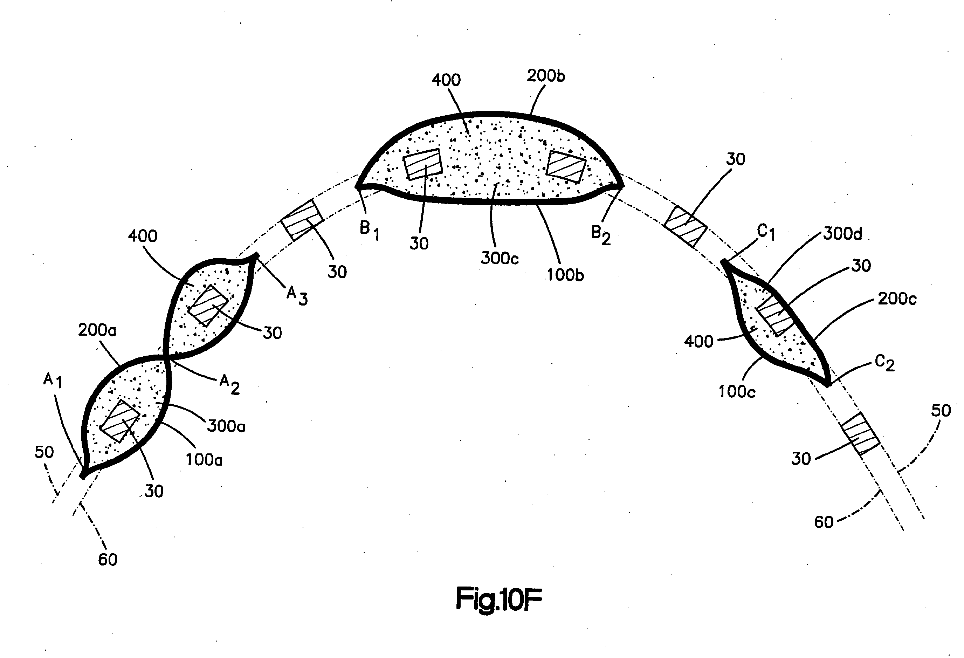

**FIG. 10F** shows another example not claimed by the invention wherein the first and second layers **100, 200** are bound to each other. Each layer **100, 200** can, but need not be bound to a strut **30.** For example, layers **100a, 200a** are bound to each other at points **A₁, A₂,** and **A₃** to form pockets **300a, 300b.** Layers **100a, 200a** are not bound to a strut **30,** however. Likewise layers **100b, 200b** are bound to each other at points **B₁ and B₂** to form pocket **300c,** which encompasses two struts **30.** Layers **100c, 200c** are bound to each other at points **C₁** and **C₂,** with layer **200c** also bound to a strut **30.** Layers **100c, 200c** form pocket **300d.**

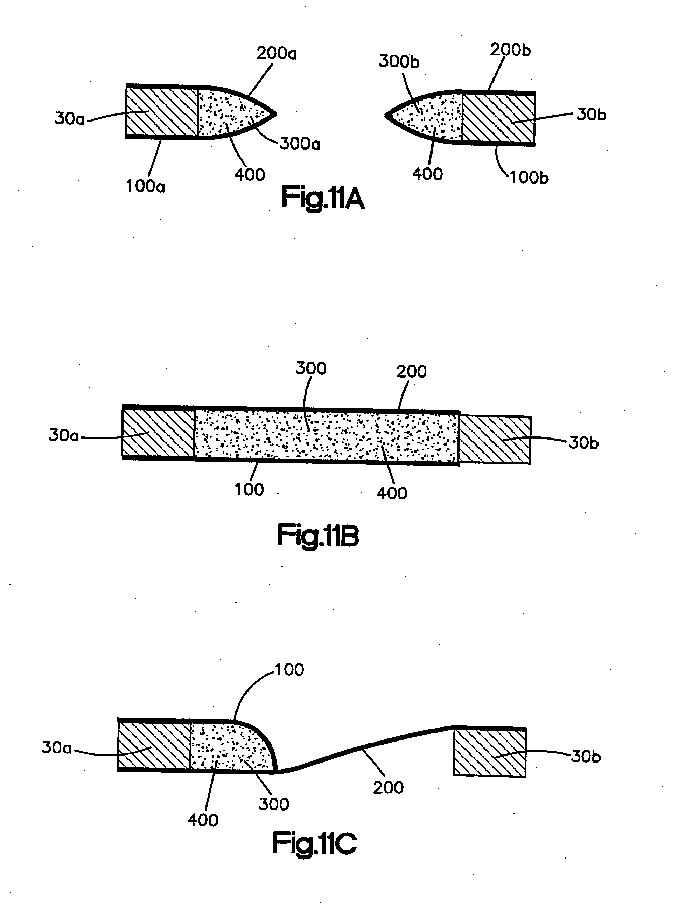

**FIGS.11A-11L** are enlarged partial cross-sectional views of pockets **300** formed from first and second layers **100, 200** disposed in openings **40** between adjacent struts **30a, 30b.** A first layer **100** may be disposed over a first sidewall and a second layer **200** may be disposed over a second sidewall. It is expressly contemplated that a pocket **300** could exhibit some or all of the characteristics of pockets described herein, and In detail below.

**FIG. 11A** shows pockets **300a, 300b not claimed by the invention formed** by first **100a, 100b** and second layers **200a, 200b,** wherein the pockets are separate from each other, and is similar to the arrangement shown in **FIG. 8O****.**

**FIG.11B** shows a pocket **300** not claimed by the invention formed by layers **100, 200,** wherein the layers contact opposite sides of adjacent strut **30a,** and terminate at the corners of strut **30b.** The embodiment shown in **FIG. 11C** shows an inverted variation similar to the embodiment of **FIG. 8F****.**

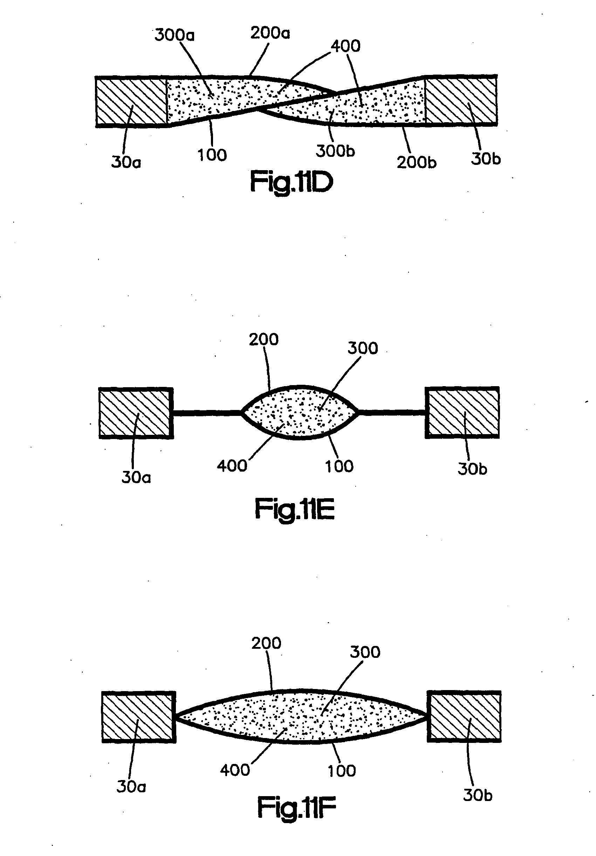

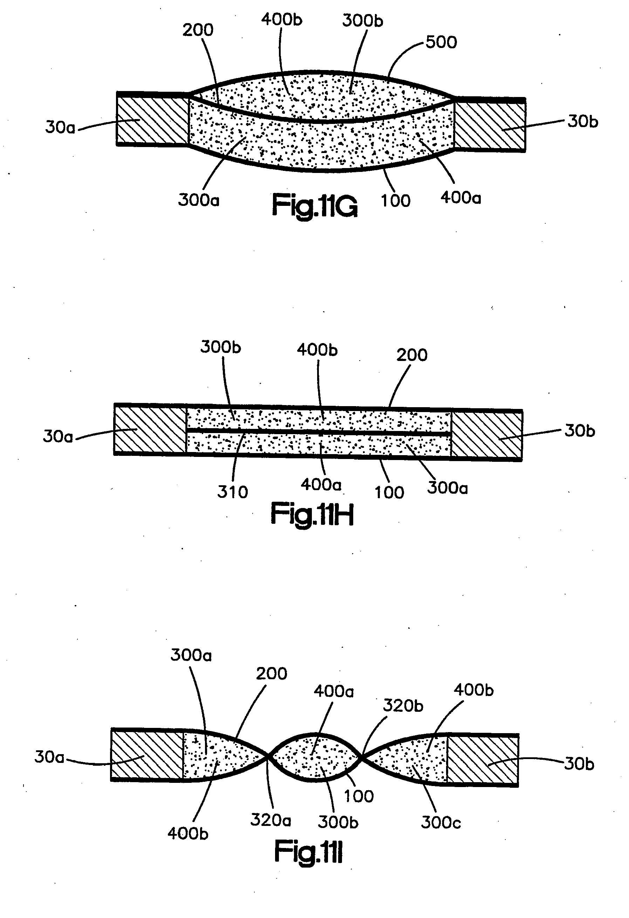

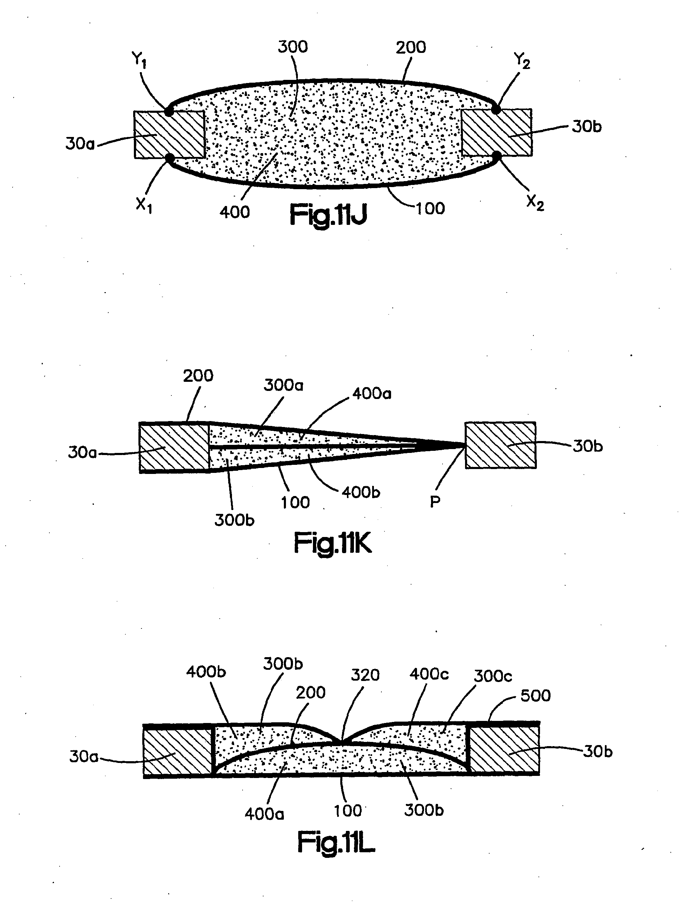

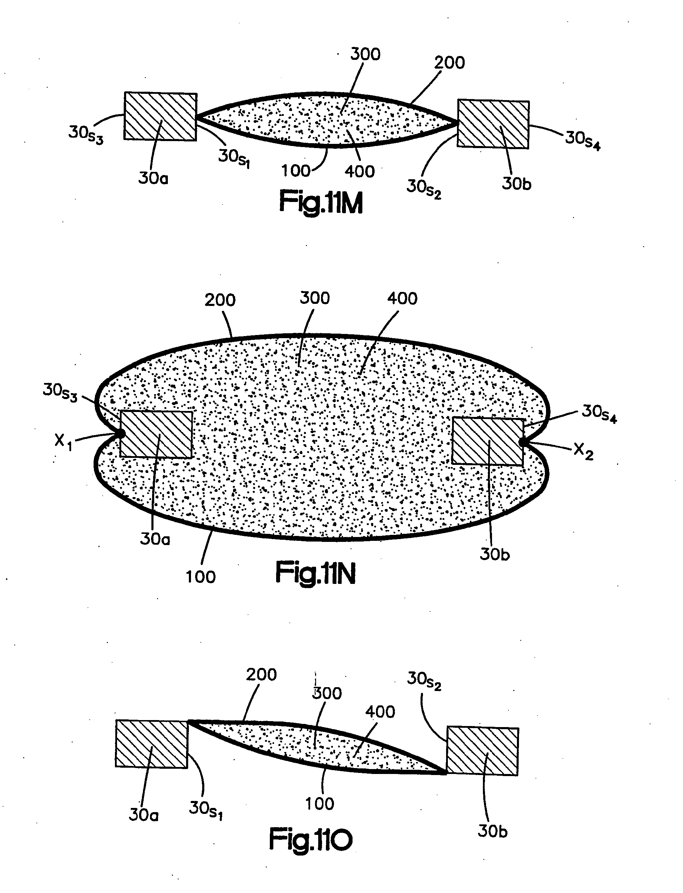

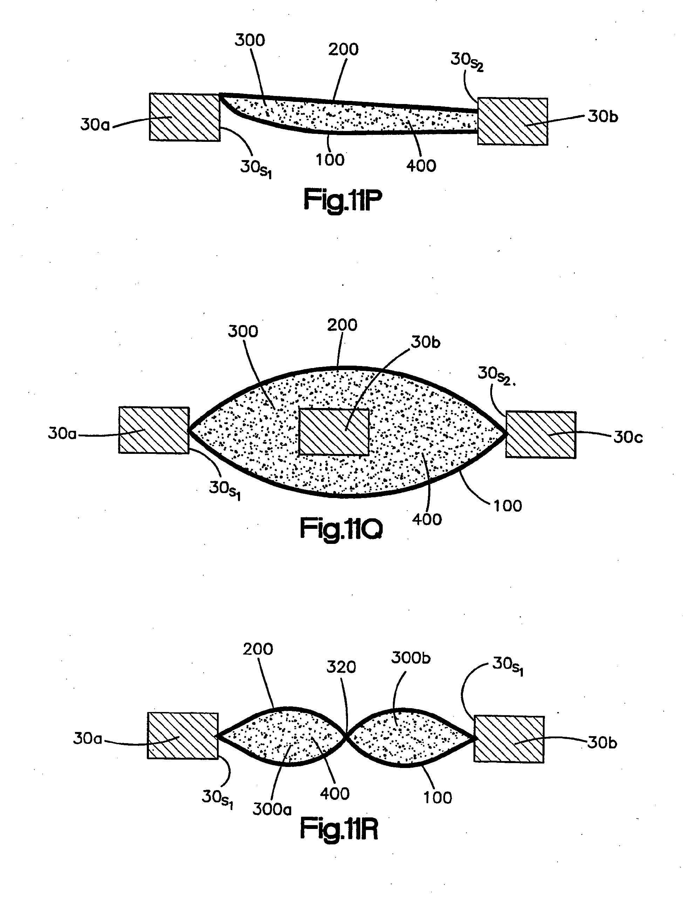

**FIG. 11** **D** shows pockets **300a, 300b** formed by running a first layer **100** through the opening **40,** and disposing second layers **200a, 200b** to form the pockets. For this particular embodiment, in addition to others, it may be beneficial to provide a first layer **100** that is relatively thicker or more resilient as compared to second layers **200a, 200b.** The characteristics of the layers are discussed in more detail, *infra.*

**FIG.11E** is a variation of the embodiments shown in **FIGS. 8B** and **8H****.** **FIG. 11F** is similar to the example of **FIG. 11E****,** wherein the pocket **300** is enlarged so that the sides of the pocket touch the struts **30a, 30b.**

**FIG. 11G** shows two "stacked" pockets **300a, 300b** formed by layers **100, 200, 500.** In this embodiment, first layer **100** is disposed along the inner sidewall surface, and second and third layers **200, 500** are disposed along the outer sidewall surface.

**FIG. 11H** shows an example similar to the arrangements shown in **FIG. 10A****,** wherein pockets **300a, 300b** are separated by barrier **310.** This example is similar to the one shown in **FIG. 8N****.**

**FIG. 11I** shows pockets in a "bowtie" arrangement, wherein layers **100, 200** are conjoined at nodes **320a, 320b** to form three pockets **300a, 300b, 300c** within the opening. In this example not claimed by the invention, pocket **300b** has a first therapeutic material **400a,** and pockets **300a** and **300c** have a second therapeutic material **400b.** Nodes **320a, 320b** may be rupturable upon expansion of stent **10,** or may remain intact as first and/or second layers **100, 200** rupture. Nodes **320a, 320b** may also be ruptured by the other methods and materials described herein.

**FIG. 11J** is a variation of **FIG. 8J****,** but wherein first and second layers **100, 200** are bonded to opposite sides of adjacent struts **30a, 30b.**

**FIG. 11K** is an example not claimed by the invention of pockets **300a, 300b** separated by barrier **310** and formed by convergent layers **100, 200.** In this arrangement, layers **100, 200** and barrier **310** all terminate at common point **P** along side surface **30s₂** of strut **30b.**

**FIG. 11L** **is** an embodiment of an opening having three pockets **300a, 300b, 300c,** containing different therapeutic materials **400a, 400b, 400c,** respectively, and centrally conjoined at node **320,** which may have any or all of the characteristics described herein.

### E. Pockets Formed by Layers Bound to Side Surfaces of Struts

In another example not claimed by the invention, pockets **300** are defined by first and second layers **100, 200** that are bound to the side surfaces of struts **30** that make-up the stent sidewall **50, 60.** In some examples, the layers are bound to the opposing side surfaces **30s₁, 30s₂** of opposing struts **30a, 30b,** such as in **FIG. 11M****.** In this example, first layer **100** and second layer **200** are each bound to side surfaces **30s₁, 30s₂** of struts **30a, 30b,** respectively. The layers **100, 200** define pocket **300** which contains a therapeutic agent **400.**

Alternatively, as shown in **FIG. 11N****,** the layers **100, 200** do not have to be bound to opposing side surfaces of struts **30,** but instead can be bound to other side surfaces **30s₃, 30s₄** of struts **30a, 30b** at points **x₁, x₂**. Similarly, the layers **100, 200** can be bound to different combinations of side surfaces.

**FIGS. 11O** and **11P** shows variations of the example of **FIG. 11M****,** wherein the layers **100, 200** are bound to different positions on the side surfaces 30s₁, 30s₂ of struts **30a, 30b.** In **FIG. 11O****,** the layers **100, 200** are bound near the top of side surface **30s₁,** but near the bottom of side surface **30s₂.** In **FIG. 11P****,** first layer **100** is bound near the top of side surface **30s₁,** and near the bottom of side surface **30s₂.** In this example not claimed by the invention, second layer **200** is bound near the top of side surfaces **30s₁, 30s₂.** As shown in these depictions, layers **100, 200** can be bound to various positions along the side surfaces of struts **30.**

**FIG. 11Q** shows an example not claimed by the invention where layers **100, 200** are not bound to adjacent struts. The pocket **300** formed in this embodiment extends beyond one opening.

**FIG. 11R** shows an example not claimed by the invention where layers **100, 200** define two pockets **300a, 300b** within a single opening.

### F. Exemplary Methods of Use and Making the Invention

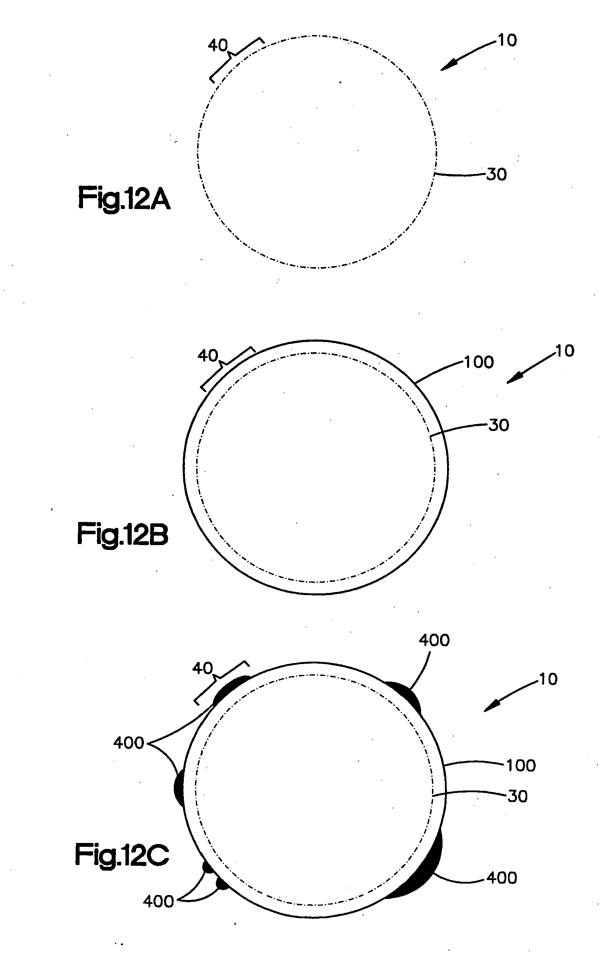

**FIGS.12A-12F** illustrate an exemplary process and use of an embodiment of a stent **10** with struts **30** and amounts of therapeutic material **400** which may be disposed within pockets **300.** In this embodiment, pockets **300** are formed by layers **100, 200** on the outer sidewall surface **60** (not shown). This method can also be used to form pockets having two layers disposed on the inner sidewall surface **50.** **FIG. 12A** shows a cross-sectional view of a stent **10** with struts **30.** Stent **10** has openings **40** between struts **30** (see, *e.g.,* **FIG. 1B****),** and is in a compressed condition. **FIG. 12B** shows the stent **10** of **FIG.12A** after an semi-flexible first layer **100** has been applied. The layers **100, 200** may be bonded to the struts **30** by using techniques known in the art, *e.g*., adhesives, heat bonding, and ultrasonic welding. The distance between the first layer **100** and the struts **30** is exaggerated to show detail and contrast.

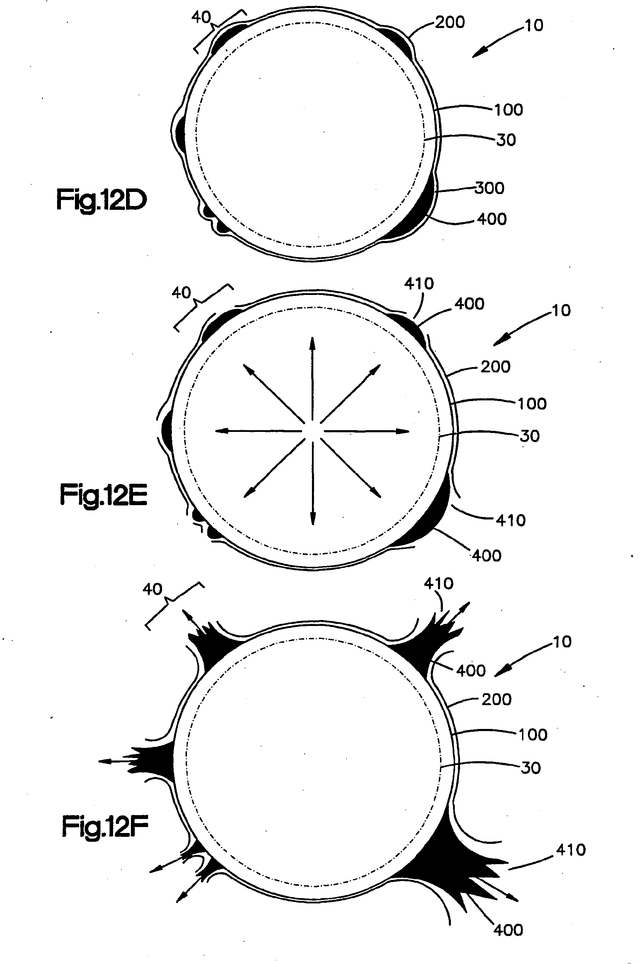

**FIG. 12C** shows the stent of **FIG. 12B** after numerous amounts of therapeutic material **400** have been applied to the stent **10** in openings **40.** As seen in **FIG. 12C****,** several sizes and shapes of amounts of therapeutic material **400** are utilized with the stent **10.** **FIG. 12D** shows the stent of **FIG. 12C** after a flexible second layer **200** has been applied, forming pockets **300.** The second layer **200** may be bonded to struts **30** and/or the first layer **100.** As seen in **FIG.12D****,** second layer **200** substantially conforms to at least some of the amounts of therapeutic material **400** within pockets **300.** The distance between the second layer **200** and the amounts of therapeutic material **400,** struts **30,** and first layer **100** has been exaggerated to show detail and contrast. **FIG. 12E** shows the stent of **FIG. 12D** in its expanded state. Struts **30,** first layer **100,** and second layer **200** have all expanded. Importantly, the second layer **200** has also ruptured, shown by ruptures **410,** at or near the locations of the amounts of therapeutic material **400** within pockets **300.** **FIG. 12F** shows the stent of **FIG. 12E** with the content of the amounts of therapeutic material **400** at least partially dispersing away from the stent **10** through ruptures **410** and toward a target site.

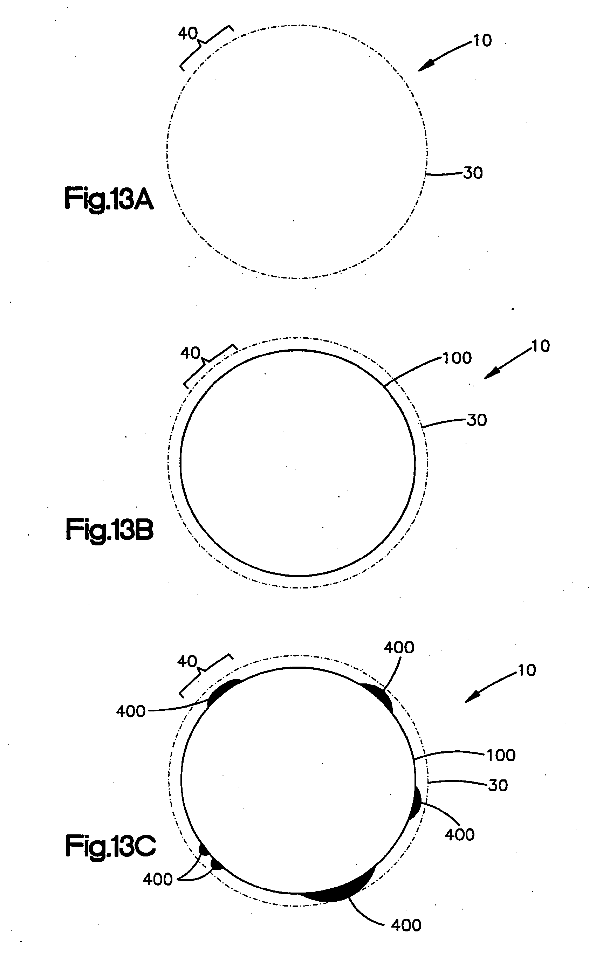

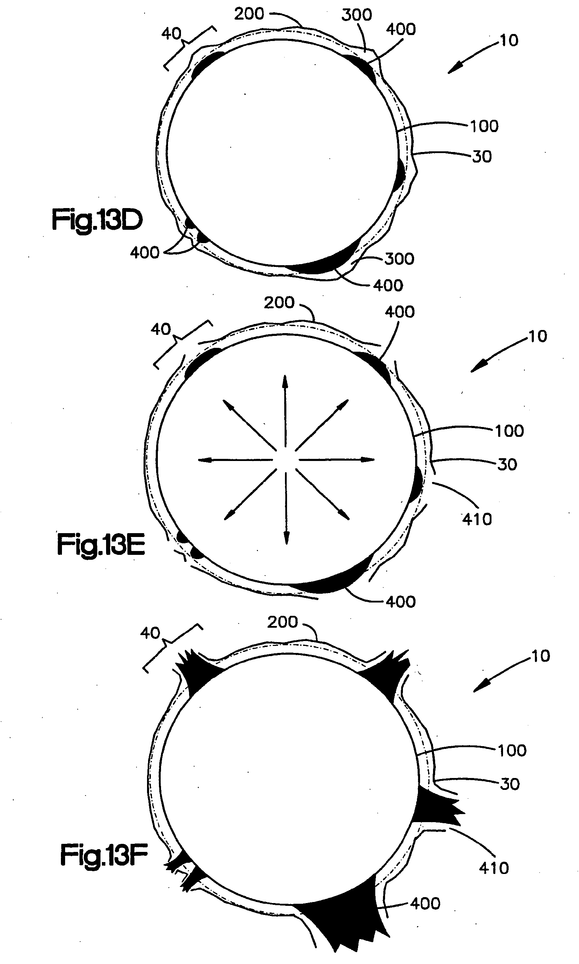

**FIGS. 13A-13F** show the exemplary method of **FIGS. 12A-12F****,** except that in this example not claimed by the invention, layers **100, 200** are disposed on different sidewall surfaces **50, 60** (not shown). The description of **FIGS. 12A-12F** applies to these figures. Also, in one example, the first and/or second layers **100, 200** can be bonded to struts **30.** In addition, in some example, the first and second layers **100, 200** are bonded to each other. Again, relative distances between objects may be exaggerated to show detail. In addition to the embodiments of method of use shown in detail, it is expressly contemplated that stent **10** may be applied with layers **100, 200** to form pockets **300** with therapeutic material **400** therein, and subsequently expanded or in some other way manipulated to release therapeutic material **400** from pockets **300.** Moreover, such a method may correspond to the numerous embodiments of pockets disclosed herein.

In the embodiment where the first and second layers **100, 200** are bound to the side surfaces of struts **30,** the pockets **300** in this embodiment may be formed by affixing the layers **100, 200** to the side surfaces using techniques known in the art. Specifically, a first layer **100** may be affixed to side surfaces of adjacent struts **30,** and then a second layer may be affixed to the surfaces of adjacent struts **30,** forming at least one pocket **300.** The layers **100, 200** can be made of materials used to make the layers **100, 200** of the other embodiments described herein.

Layers **100, 200** may also be applied to a stent **10** in the form of a polymer slurry, which after application to at least a portion of the stent **10,** may be allowed to dry and/or cured and form a layer **100, 200** on the stent **10.** Layer **100, 200** thickness may be varied by altering the polymer slurry consistency, dip rate, and or curing conditions. A slurry may be applied to the stent 10 in the expanded or unexpanded state.

### G. Further Embodiments of Struts

FIGS. **14A-14D** are enlarged partial cross-sectional views of pockets **300** formed from first and second layers **100, 200** disposed in openings **40** between adjacent struts **30a, 30b.** It is expressly contemplated that a pocket **300** could exhibit some or all of the characteristics of pockets described herein, and in detail below. More specifically, **FIGS. 14A-14D** are exemplary strut shapes and sizes for use with the present invention.

**FIG. 14A** shows an embodiment where struts **30a, 30b** have a rounded cross-sectional shape. Such a shape may be circular, elliptical, oval, or some combination thereof. **FIG. 14B** shows an example wherein a rectangular strut **30a** is paired with a rounded strut **30b.** **FIG. 14C** shows another embodiment of struts, wherein the struts **30a, 30b** are hexagonal. Further suitable cross-sectional strut shapes include squares, parallelograms, triangles, octagons, irregular shapes, or any other polygonal shape. It is further noted that any combination of suitable shapes may be used on a single stent **10.**

**FIG. 14D** shows an example wherein adjacent struts **30a, 30b** are of substantially different size. Such size variation may be used with any combination of shapes discussed herein, or that may be appreciated by those skilled in the art.

Overall, it is expressly contemplated that the pocket and layering designs shown and described in reference to the figures herein may be varied and/or combined by those skilled in the art. The designs shown are exemplary and the concepts and variations shown are intended to be viewed as several of the many examples contemplated by providing struts and layers to form pockets **300.**

### H. The Layers

Layers **100, 200** may be composed of one or more sub-layers (not shown). Layers **100, 200** may be comprised of a variety of suitable materials, such as Polyurethane or Silicone, or a suitable polymer. More than one material may be used for individual sub-layers to create a first or second layer **100, 200.** First and second layer **100, 200** may be comprised of different materials. Having the first and second layers **100, 200** different materials may also a user to vary the porosity, tear strength, breakdown rate, and/or texture of each layer individually. The selection and variance of these attributes may be beneficial if, for example, it is desirable that the contents of a pocket **300** (such as an amount of therapeutic material **400)** are to be delivered through the second layer **200,** but preferably not the first layer **100.** It may also be desirable to alter the release rates of the contents of a pocket 300 based upon the choice and/or combination of materials and methods used in applying each layer **100, 200** to a stent **10.** For instance, the chosen material for a layer may be relatively porous, to allow the contents of the pocket **300** to disperse slowly. A detailed discussion of suitable materials for layers **100, 200** appears below.

The chosen material for each layer may be applied to the stent **10** while in the form of a slurry. Layers **100, 200** may be directly applied to a stent **10** by dispensing a slurry to the stent, or by affixing the stent onto a cylindrical mandrel and dipping the assembly into a slurry. The thickness of each sub-layer or layer may be altered based on the consistency of the slurry, the dipping rate, and/or the curing conditions. Other methods and materials for applying layers to the stent may be utilized as deemed appropriate by one skilled in the art.

Layers **100, 200** may be applied while the stent **10** is in its collapsed or expanded state. If the layers are applied to the stent **10** while the stent is in its collapsed state, the layers should be comprised at least in part of a flexible material that is able to stretch when the stent **10** expands. A layer that is inflexible may undesirably rupture upon the expansion of the stent **10.**

Varying flexibility of a layer may also allow for increased or decreased capabilities in pocket volume and dimensions. For example, if the first layer **100** is made of a more rigid material, and the second layer **200** is made of a more flexible material, the pocket may tend to "bulge" outwards, utilizing the increased flexibility of the second layer **200.** Such an arrangement may be preferable when it is desirable to maintain the first shape of the stent **10** to, for example, maintain a maximum flow path therethrough. Moreover, it may be preferable to create or supplement a pocket 300 after the layer is complete, by such means as a injecting element. Having at least one layer **100, 200** made of a flexible material may allow a increased amount of content to be inserted into a pocket, as the pocket could "stretch" to increase its volume as its is filled.

Furthermore, it may also be preferable to have increased flexibility with the first layer **100** to pattern the rupture and/or dispersion of the content of the pockets **300** to the inside of the stent **10.** This may be assisted by the expansion of a balloon (not shown) inside the stent, the pressure of which against the first layer **100** could cause ruptures and allow for dispersion of the content of the pockets **300** along the inside of the stent **10.**

The layers **100, 200** may also be made of a biodegradable material. Similarly, it may be preferable for first and second layer to have varying degrees of biodegradability to assist in controlling the release rate of the content of a pocket.

The layers **100, 200** may also be applied to the stent with pre-cut tears in the layer. The first and/or second layers may have such tears. When the stent expands, as seen in **FIGS. 12E****,** **13E****,** the tears may localize the points at which a layer ruptures. Therefore, a user may exert increased control over the dispersion pattern and area of the content of the pockets **300** by preselecting the tear and rupture points of layer. Such a design may be especially desirable when the target tissue site for content delivery is very localized, or it is undesirable to deliver the content to areas other than the target site, such as the bloodstream.

As an alternative to making pre-cut tears in a layer to dictate rupture points, a layer may be imprinted by used of a contour mandrel during the layering process. The surface of such a contoured mandrel may not create punctures or tears in the layer upon formation, but instead would imprint patterns of thinner or weaker areas in the layer. Upon expansion, the imprinted areas would preferably be the first areas to rupture.

When using a first layer **100** that is not entirely rigid, it may also be desirable to expand the pockets **300** towards the longitudinal axis of a hollow cylindrical stent. This may be accomplished by simply providing a flexible first layer. The expansion of the pockets 300 toward the longitudinal axis may also be urged by using a hollow cylindrical mandrel, having a longitudinal axis substantially coaxial to the axis, to apply the first layer and subsequently running a vacuum through the mandrel to exert an axial force on the pockets 300, pulling them toward the longitudinal axis.

To assist or cause the rupture of the first and/or second layers, it may also be preferable to place a spike (or other equivalent sharpened element) within a void or pocket to puncture the first and/or second layers when the stent **100** is expanded. The spike may be bioresorbable, and/or may also be part of the strut **30** structure itself. A related embodiment is to provide spikes (or other equivalent sharpened elements) on the balloon itself. When the balloon expands, the spikes on the balloon would then puncture the first and/or second layers, allowing the content of the affected pockets **300** to disperse. Such balloons are known in the art as infiltrating balloons or cutting balloons. When using such a balloon, it may be preferable to make the first layer **100** and/or second layer **200** of a self-sealing material, to enable the first layer to close its punctures after the balloon has retracted.

As an alternative to using a balloon or other expanding device to rupture pockets **300** of a stent **10,** pockets may be ruptured locally by the use of an ultrasonic device. In such an embodiment, the pockets **300** could have therapeutically-loaded microbubbles which would burst in response to an ultrasonic impetus.

Stent **10** may also be ruptured by way of a time-delayed decay. In such an embodiment, at least one layer would be at least partially comprised of a biodegradable material, which would be configured to decay over a predetermined period of time to eventually release a therapeutic agent.

More than one stent **10** may also be arranged in a combination or matrix format. Such uses of more than one stent **10** are known in the art.

It should be noted as well that the use of layers with a stent may also be beneficial in protecting the contents of the pocket, the stent itself, and any expansive device (such as a balloon) during the implantation of the assembly into the body. Stents directly coated with therapeutic agents can lose significant quantities of their agent during implantation, as the stent will often come into contact with vessel walls, bodily fluids, etc. before reaching the target site. The use of layers over the pockets may help guard against such a loss of therapeutic material.

### I. Therapeutic Agents

The contents of a pocket **300** and/or coating may contain one or more biological active materials, such as an amount of therapeutic material **400.** The term "biologically active material" encompasses therapeutic agents, such as biologically active agents, and also genetic materials and biological materials. The term "therapeutic agent" as used in the present invention encompasses drugs, genetic materials, and biological materials and can be used interchangeably with "biologically active material". Non-limiting examples of suitable therapeutic agent include heparin, heparin derivatives, urokinase, dextrophenylalanine proline arginine chloromethylketone (PPack), enoxaprin, angiopeptin, hirudin, acetylsalicylic acid, tacrolimus, everolimus, rapamycin (sirolimus), amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti-thrombin antibodies, anti-platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, antioxidants, probucol, antibiotic agents, penicillin, cefoxitin, oxacillin, tobranycin, angiogenic substances, fibroblast growth factors, estrogen, estradiol (E2), estriol (E3), 17-beta estradiol, digoxin, beta blockers, captopril, enalopril, statins, steroids, vitamins, taxol, paclitaxel, 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In one embodiment, the therapeutic agent is a smooth muscle cell inhibitor or antibiotic. In a preferred embodiment, the therapeutic agent is taxol (*e.g*., Taxol®), or its analogs or derivatives. In another preferred embodiment, the therapeutic agent is paclitaxel, or its analogs or derivatives. In yet another preferred embodiment, the therapeutic agent is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g*., BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (*e.g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (*e.g*., RGD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g*., mesenchymal, hematopoietic, neuronal), stromal cells, parenchymal cells, undifferentiated cells, fibroblasts, macrophage, and satellite cells.

Other non-genetic therapeutic agents include:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents, vasodilating agents, and agents which interfere with endogenous vasoactive mechanisms;
- anti-oxidants, such as probucol;
- antibiotic agents, such as penicillin, cefoxitin, oxacillin, tobranycin, rapamycin (sirolimus);
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-converting enzyme (ACE) inhibitors including captopril and enalopril, statins and related compounds; and
- macrolides such as sirolimus or everolimus.

Preferred biological materials include anti-proliferative drugs such as steroids, vitamins, and restenosis-inhibiting agents. Preferred restenosis-inhibiting agents include microtubule stabilizing agents such as Taxol®, paclitaxel (*i.e*., paclitaxel, paclitaxel analogs, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt.

Other suitable therapeutic agents include tacrolimus; halofuginone; inhibitors of HSP90 heat shock proteins such as geldanamycin; microtubule stabilizing agents such as epothilone D; phosphodiesterase inhibitors such as cliostazole; Barkct inhibitors; phospholamban inhibitors; and Serca 2 gene/proteins.

Other preferred therapeutic agents include nitroglycerin, nitrous oxides, nitric oxides, aspirins, digitalis, estrogen derivatives such as estradiol and glycosides.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices of the present invention can be synthesized by methods well known to one skilled in the art. Alternatively, the therapeutic agents can be purchased from chemical and pharmaceutical companies.

The solvent that is used to form the coating composition include ones which can dissolve the polymer into solution and do not alter or adversely impact the therapeutic properties of the therapeutic agent employed. Examples of useful solvents include tetrahydrofuran (THF), methyl ethyl ketone chloroform, toluene, acetone, issoctane, 1,1,1-trichloroethane, isoppropanol, IPA and dichloromethane or mixtures thereof.

### J. Coating the Stent

It may be beneficial to apply a coating to a stent **10** with pockets **300.** The coating can be applied over the layers **100, 200** forming pockets **300,** and/or over parts of the stent **10** that are not covered by a layer **100, 200.** A coating composition may be prepared, for example, by applying a mixture of a polymeric material, a solvent and a therapeutic agent on a surface to form a coating. If such a composition is used the polymeric material incorporates the therapeutic agent. Alternatively, the coating composition may not include a polymeric material. The following is a description of suitable materials and methods useful in producing a coating on the surface of stent struts of the invention.

Polymeric materials useful for forming the coating should be ones that are biocompatible, particularly during insertion or implantation of the device into the body and avoids irritation to body tissue. Examples of such polymers include, but not limited to, polyurethanes, polyisobutylene and its copolymers, silicones, and polyesters. Other suitable polymers include polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate; copolymers of vinyl monomers, copolymers of vinyl monomers and olefins such as ethylene-methyl methacrylate copolymers, acrylonitrile-styrene copolymers, ABS resins, ethylene-vinyl acetate copolymers, polyamides such as Nylon 66 and polycaprolactone, alkyd resins, polycarbonates, polyoxyethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, and polylactic acid-polyethylene oxide copolymers. Since the polymer is being applied to a part of the medical device which undergoes mechanical challenges, *e.g.* expansion and contraction, the polymers are preferably selected from elastomeric polymers such as silicones (*e.g*. polysiloxanes and substituted polysiloxanes), polyurethanes, thermoplastic elastomers, ethylene vinyl acetate copolymers, polyolefin elastomers, and EPDM rubbers. The polymer is selected to allow the coating to better adhere to the surface of the strut when the stent is subjected to forces or stress. Furthermore, although the coating can be formed by using a single type of polymer, various combinations of polymers can be employed.

Generally, when a biologically active material used is a hydrophilic, *e.g.,* heparin, then a matrix material comprising a more hydrophilic material has a greater affinity for the biologically active material than another matrix material that is less hydrophilic. When a biologically active material used is a hydrophobic, *e.g.*, paclitaxel, actinomycin, sirolimus (RAPAMYCIN), tacrolimus, everolimus, and dexamethasone, then a matrix material that is more hydrophobic has a greater affinity for the biologically active material than another matrix material that is less hydrophobic.

Examples of suitable hydrophobic polymers include, but not limited to, polyolefins, such as polyethylene, polypropylene, poly(1-butene), poly(2-butene), poly(1-pentene), poly(2-pentene), poly(3-methyl-1-pentene), poly(4-methyl-1-pentene), poly(isoprene), poly(4-methyl-1-pentene), ethylene-propylene copolymers, ethylene-propylene-hexadiene copolymers, ethylene-vinyl acetate copolymers, blends of two or more polyolefins and random and block copolymers prepared from two or more different unsaturated monomers; styrene polymers, such as poly(styrene), poly(2-methylstyrene), styrene-acrylonitrile copolymers having less than about 20 mole-percent acrylonitrile, and styrene-2,2,3,3,-tetrafluoropropyl methacrylate copolymers; halogenated hydrocarbon polymers, such as poly(chlorotrifluoroethylene), chlorotrifluoroethylene-tetrafluoroethylene copolymers, poly(hexafluoropropylene), poly(tetrafluoroethylene), tetrafluoroethylene, tetrafluoroethylene-ethylene copolymers, poly(trifluoroethylene), poly(vinyl fluoride), and poly(vinylidene fluoride); vinyl polymers, such as poly(vinyl butyrate), poly(vinyl decanoate), poly(vinyl dodecanoate), poly (vinyl hexadecanoate), poly(vinyl hexanoate), poly(vinyl propionate), poly(vinyl octanoate), poly(heptafluoroisopropoxyethylene), poly(heptafluoroisopropoxypropylene), and poly(methacrylonitrile); acrylic polymers, such as poly(n-butyl acetate), poly(ethyl acrylate), poly(1-chlorodifluoromethyl)tetrafluoroethyl acrylate, poly di(chlorofluoromethyl)fluoromethyl acrylate, poly(1,1-dihydroheptafluorobutyl acrylate), poly(1,1-dihydropentafluoroisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate, and poly(nonafluoroisobutyl acrylate); methacrylic polymers, such as poly(benzyl methacrylate), poly(n-butyl methacrylate), poly(isobutyl methacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poly(heptadecafluorooctyl methacrylate), poly(1-hydrotetrafluoroethyl methacrylate), pofy(1,1-dihydrotetrafluoropropyl methacrylate), poly(1-hydrohexafluoroisopropyl methacrylate), and poly(t-nonafluorobutyl methacrylate); polyesters, such a poly(ethylene terephthalate) and poly(butylene terephthalate); condensation type polymers such as and polyurethanes and siloxane-urethane copolymers; polyorganosiloxanes, *i.e*., polymeric materials characterized by repeating siloxane groups, represented by Rₐ SiO _{4-a/2}, where R is a monovalent substituted or unsubstituted hydrocarbon radical and the value of a is 1 or 2; and naturally occurring hydrophobic polymers such as rubber.

Examples of suitable hydrophilic monomer include, but not limited to; (meth)acrylic acid, or alkaline metal or ammonium salts thereof; (meth)acrylamide; (meth)acrylonitrile; those polymers to which unsaturated dibasic, such as maleic acid and fumaric acid or half esters of these unsaturated dibasic acids, or alkaline metal or ammonium salts of these dibasic adds or half esters, is added; those polymers to which unsaturated sulfonic, such as 2-acrylamido-2-methylpropanesulfonic, 2-(meth)acryloylethanesulfonic acid, or alkaline metal or ammonium salts thereof, is added; and 2-hydroxyethyl (meth)acrylate and 2-hydroxypropyl (meth)acrylate.

Polyvinyl alcohol is also an example of hydrophilic polymer. Polyvinyl alcohol may contain a plurality of hydrophilic groups such as hydroxyl, amido, carboxyl, amino, ammonium or sulfonyl (-SO₃). Hydrophilic polymers also include, but are not limited to, starch, polysaccharides and related cellulosic polymers; polyalkylene glycols and oxides such as the polyethylene oxides; polymerized ethylenically unsaturated carboxylic acids such as acrylic, mathacrylic and maleic acids and partial esters derived from these acids and polyhydric alcohols such as the alkylene glycols; homopolymers and copolymers derived from acrylamide; and homopolymers and copolymers of vinylpyrrolidone.

Suitable stents may also be coated or made with non-polymeric materials. Examples of useful non-polymeric materials include sterols such as cholesterol, stigmasterol, β-sitosterol, and estradiol; cholesteryl esters such as cholesteryl stearate; C₁₂-C₂₄ fatty acids such as lauric acid, myristic acid, palmitic acid, stearic acid, arachidic acid, behenic acid, and lignoceric acid; C₁₈ -C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; C₁₆ -C₁₈ fatty alcohols such as cetyl alcohol, myristyl alcohol, stearyl alcohol, and cetostearyl alcohol; esters of fatty alcohols and fatty acids such as cetyl palmitate and cetearyl palmitate; anhydrides of fatty acids such as stearic anhydride; phospholipids including phosphatidylcholine (lecithin), phosphatidylserine, phosphatidylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymeric materials include cholesterol, glyceryl monostearate, glycerol tristearate, stearic acid, stearic anhydride, glyceryl monooleate, glyceryl monolinoleate, and acetylated monoglycerides.

Coating compositions can be applied by any method to a surface of a medical device to form a coating layer. Examples of suitable methods include, but are not limited to, spraying such as by conventional nozzle or ultrasonic nozzle, dipping, rolling, electrostatic deposition, and a batch process such as air suspension, pan coating or ultrasonic mist spraying. Also, more than one coating method can be used to make a medical device. Coating compositions suitable for applying a coating to the devices of the present invention can include a polymeric material dispersed or dissolved in a solvent suitable for the medical device, wherein upon applying the coating composition to the medical device, the solvent is removed. Such systems are commonly known to the skilled artisan.

A coating of a medical device of the present invention may include multiple coating layers. For example, the first layer and the second layer may contain different biologically active materials. Alternatively, the first layer and the second layer may contain an identical biologically active material having different concentrations. In one embodiment, either of the first layer or the second layer may be free of biologically active material. For example, when the biologically active solution is applied onto a surface and dried (the first layer), a coating composition free of a biologically active material (the second layer) can be applied over the dried biologically active material.

The description contained herein is for purposes of illustration and not for purposes of limitation. Changes and modifications may be made to the embodiments of the description and still be within the scope of the invention which is defined by the features of the appended claims.

While the invention has been shown and described herein with reference to particular embodiments, it is to be understood that the various additions, substitutions, or modifications of form, structure, arrangement, proportions, materials, and components and otherwise, used in the practice and which are particularly adapted to specific environments and operative requirements, may be made to the described embodiments without departing from the scope of the present invention which is defined by the features of the appended claims. Accordingly, it should be understood that the embodiments disclosed herein are merely illustrative of the principles of the invention.

## Claims

1. A medical device for delivering a therapeutic agent (400, 400a, 400b) comprising:
(a) a stent (10) having a sidewall (20) comprising a plurality of struts (30, 30a, 30b) and at least a first opening (40) in the sidewall, the first opening (40) being between adjacent struts (30, 30a, 30b), the sidewall having a first sidewall surface (50, 60), wherein the first sidewall surface (50, 60) is either an outer sidewall surface (60), which faces a body lumen wall when the stent (10) is implanted, or an inner sidewall surface (50), which faces away from the body lumen wall;
(b) a first layer (100) disposed over at least a part of the first sidewall surface (50, 60), wherein at least a portion of the first layer (100) extends over a part of the first opening (40);
(c) a second layer (200) disposed over at least a part of the first sidewall surface (50, 60), wherein at least a portion of the second layer (200) is disposed over the portion of the first layer (100) that extends over the first opening (40),
(d) at least one first pocket (300, 300a, 300c, 300d, 300e) disposed about at least a portion of the first opening (40); at least a portion of the first pocket (300, 300a, 300c, 300d, 300e) extending into the first opening (40), wherein the pocket is defined at least in part by a portion of the first layer (100), a portion of the second layer (200), and by a first bond between the first layer (100) and the second layer (200); and
(e) a therapeutic agent (400, 400a, 400b) contained in the first pocket (300, 300a, 300c, 300d, 300e).

2. The medical device of claim 1, wherein the first layer (100) is bound to the stent (10).

3. The medical device of claim 1, wherein the second layer (200) is bound to the stent (10).

4. The medical device of claim 1, wherein the first pocket (300, 300a, 300c, 300d, 300e) is co-extensive with the first opening (40).

5. The medical device of claim 1, wherein the first pocket (300, 300a, 300c, 300d, 300e) is within the first opening (40).

6. The medical device of claim 1, wherein the first pocket (300, 300a, 300c, 300d, 300e) extends beyond the first opening (40).

7. The medical device of claim 1, further comprising a second pocket disposed about the first opening (40).

8. The medical device of claim 1, wherein at least one strut (30, 30a, 30b) comprises a side surface (30ₛ₁, 30ₛ₂) and the first layer (100) is disposed over the side surface.

9. The medical device of claim 1, wherein at least one strut (30, 30a, 30b) comprises a side surface (30ₛ₁, 30ₛ₂) and the second layer (200) is disposed over the side surface.

10. The medical device of claim 1, wherein at least one strut (30, 30a, 30b) comprises a side surface (30ₛ₁, 30ₛ₂) and the first and second layers (100, 200) are disposed over the side surface.

11. The medical device of claim 1, wherein the sidewall (20) further comprises a second sidewall surface (50, 60) and a third layer is disposed over the second sidewall surface (50, 60).

12. The medical device of claim 11, wherein the sidewall (20) further comprises a fourth layer disposed over at least a portion of the third layer.

13. The medical device of claim 1, wherein the stent (10) further comprises a second opening, and wherein a second pocket is disposed about the second opening.

14. The medical device of claim 13, wherein the second pocket is defined at least in part by the first layer (100) and at least in part by the second layer (200).

15. The medical device of claim 13, wherein the second pocket is defined at least in part by a third layer and at least in part by a fourth layer.

16. The medical device of claim 15, wherein the third layer is disposed over at least a portion of the first sidewall surface (50, 60), and the fourth layer is disposed over at least a portion of the second sidewall surface (50, 60).

17. The medical device of claim 13, wherein the second pocket contains a therapeutic agent (400, 400a, 400b).

18. The medical device of claim 1, further comprising a second pocket, and wherein the second pocket contains a different therapeutic agent (400, 400a, 400b) than the first pocket (300, 300a, 300c, 300d, 300e).

19. The medical device of claim 18, wherein the second pocket is disposed about the first opening (40).

20. The medical device of claim 18, further comprising a second pocket (300, 300a, 300c, 300d, 300e), and wherein the first and second pockets (300, 300a, 300c, 300d, 300e) are interconnected.

21. The medical device of claim 1, wherein at least one of the first and second layers (100, 200) comprises a plurality of sub-layers.

22. The medical device of claim 21, wherein at least two sub-layers are comprised of a different material.

23. The medical device of claim 21, wherein at least two sub-layers are of different thicknesses.

24. The medical device of claim 1, further comprising a barrier (310) between the first and second layers (100, 200).

25. The medical device of claim 1, further comprising a third layer disposed over at least a portion of one of the first and second layers (100, 200).

26. The medical device of claim 1, wherein the first and second layers (100, 200) are comprised of the same material.

27. The medical device of claim 1, wherein the first and second layers (100, 200) are comprised of different materials.

28. The medical device of claim 1, wherein the first and second layers (100, 200) have different tensile strengths.

29. The medical device of claim 1, wherein the first and second layers (100, 200) are of different thicknesses.

30. The medical device of claim 1, wherein at least one of the first and second layer (100, 200) is capable of being ruptured by the expansion of the stent (10).

31. The medical device of claim 1, wherein at least a portion of at least one of the first and second layer (100, 200) comprises a plurality of pores.

32. The medical device of claim 1, wherein at least one of the first layer (100) and second layer (200) comprise at least one preformed imprint, and wherein the imprinted area generally have a lower tensile strength than the remainder of the layer.

33. The medical device of claim 1, wherein at least one of the first layer (100) and second layer (200) comprises a self-sealing material.

34. The medical device of claim 1, wherein at least one of the first layer (100) and second layer (200) comprises a biodegradable material.

35. The medical device of claim 1, wherein at least one of the first layer (100) and second layer (200) are substantially flexible.

36. The medical device of claim 1, wherein the therapeutic agent (400, 400a, 400b) is releasable from the first pocket (300, 300a, 300c, 300d, 300e) through at least one of the first layer (100) and second layer (200).

37. The medical device of claim 1, wherein the therapeutic agent (400, 400a, 400b) is releasable from the first pocket (300, 300a, 300c, 300d, 300e) after the expansion of the stent (10).

## Patentansprüche

1. Eine medizinische Vorrichtung zum Zuführen eines Therapeutikums (400, 400a, 400b) umfassend:
(a) einen Stent (10) mit einer Seitenwand (20) umfassend eine Mehrzahl von Streben (30, 30a, 30b) und mindestens eine erste Öffnung (40) in der Seitenwand, wobei die erste Öffnung (40) zwischen benachbarten Streben (30, 30a, 30b) ist, die Seitenwand eine erste Seitenwand-Oberläche (50, 60) hat, wobie die erste Seitenwand-Oberfläche (50, 60) entweder eine äußere Seitenwand-Oberfläche (60) ist, die zu einer Körperlumen-Wand zeigt, wenn der Stent (10) implantiert ist, oder eine innere Seitenwand-Oberfläche (50), die von der Körperlumen-Wand wegzeigt;
(b) eine erste Schicht (100) angeordnet über zumindest einem Teil der ersten Seitenwand-Oberfläche (50, 60), wobei sich zumindest ein Teil der ersten Schicht (100) über einen Teil der ersten Öffnung (40) erstreckt;
(c) eine zweite Schicht (200) angeordnet über zumindest einem Teil der ersten Seitenwand-Oberfläche (50, 60), wobei zumindest ein Teil der zweiten Schicht (200) über dem Teil der ersten Schicht (100) angeordnet ist, der sich über die erste Öffnung (40) erstreckt;
(d) zumindest eine erste Tasche (300, 300a, 300c, 300d, 300e) angeordnet über zumindest einen Teil der ersten Öffnung (40); zumindest ein Teil der ersten Tasche (300, 300a, 300c, 300d, 300e) erstreckt sich in die erste Öffnung (40), wobei die Tasche zumindest teilweise definiert ist durch einen Teil der ersten Schicht (100), einen Teil der zweiten Schicht (200), und durch eine erste Verbindung zwischen der ersten Schicht (100) und der zweiten Schicht (200); und
(e) einem Therapeutikum (400, 400a, 400b) enthalten in der ersten Tasche (300, 300a, 300c, 300d, 300e).

2. Die medizinische Vorrichtung gemäß Anspruch 1, wobei die erste Schicht (100) an den Stent (10) gebunden ist.

3. Die medizinische Vorrichtung gemäß Anspruch 1, wobei die zweite Schicht (200) an den Stent (10) gebunden ist.

4. Die medizinische Vorrichtung gemäß Anspruch 1, wobei die erste Tasche (300, 300a, 300c, 300d, 300e) flächengleich mit der ersten Öffnung (40) ist.

5. Die medizinische Vorrichtung gemäß Anspruch 1, wobei die erste Tasche (300, 300a, 300c, 300d, 300e) innerhalb der ersten Öffnung (40) ist.

6. Die medizinische Vorrichtung gemäß Anspruch 1, wobei die erste Tasche (300, 300a, 300c, 300d, 300e) sich über die erste Öffnug (40) hinaus erstreckt.

7. Die medizinische Vorrichtung gemäß Anspruch 1, weiter umfassend eine zweite Tasche angeordnet über der ersten Öffnung (40).

8. Die medizinische Vorrichtung gemäß Anspruch 1, wobei zumindest eine Strebe (30, 30a, 30b) eine Steitenfläche (30_{S1}, 30_{S2}) umfasst und die erste Schicht (100) über der Seitenfläche angeordnet ist.

9. Die medizinische Vorrichtung gemäß Anspruch 1, wobei zumindest eine Strebe (30, 30a, 30b) eine Steitenfläche (30_{S1}, 30_{S2}) umfasst und die zweite Schicht (200) über der Seitenfläche angeordnet ist.

10. Die medizinische Vorrichtung gemäß Anspruch 1, wobei zumindest eine Strebe (30, 30a, 30b) eine Steitenfläche (30_{S1}, 30_{S2}) umfasst und die ersten und zweiten Schichten (100, 200) über der Seitenfläche angeordnet sind.

11. Die medizinische Vorrichtung gemäß Anspruch 1, wobei die Seitenwand (20) weiter umfasst eine zweite Seitenwand-Oberfläche (50, 60) und eine dritte Schicht über der zweiten Seitenwand-Oberfläche (50, 60) angeordnet ist.

12. Die medizinische Vorrichtung gemäß Anspruch 11, wobei die Seitenwand (20) weiter umfasst eine vierte Schicht, die zumindest über einem Teil der dritten Schicht angeordnet ist.

13. Die medizinische Vorrichtung gemäß Anspruch 1, wobei der Stent (10) weiter umfasst eine zweite Öffnung, und wobei eine zweite Tasche über der zweiten Öffnung angeordnet ist.

14. Die medizinische Vorrichtung gemäß Anspruch 13, wobei die zweite Tasche definiert ist zumindest teilweise durch die erste Schicht (100) und zumindest teilweise durch die zweite Schicht (200).

15. Die medizinische Vorrichtung gemäß Anspruch 13, wobei die zweite Tasche definiert ist zumindest teilweise durch eine dritte Schicht und zumindest teilweise duch eine vierte Schicht.

16. Die medizinische Vorrichtung gemäß Anspruch 15, wobei die dritte Schicht zumindest über einem Teil der ersten Seitenwand-Oberfläche (50, 60) angeordnet ist, und die vierte Schicht zumindest über einem Teil der zweiten Seitenwand-Oberlfäche (50, 60) angeordnet ist.

17. Die medizinische Vorrichtung gemäß Anspruch 13, wobei die zweite Tasche ein Therapeutikum (400, 400a, 400b) enthält.

18. Die medizinische Vorrichtung gemäß Anspruch 1, weiter umfassend eine zweite Tasche, und wobei die zweite Tasche ein anderes Therapeutikum (400, 400a, 400b) als die erste Tasche (300, 300a, 300c, 300d, 300e) enthält.

19. Die medizinische Vorrichtung gemäß Anspruch 18, wobei die zweite Tasche über der ersten Öffnung (40) angeordnet ist.

20. Die medizinische Vorrichtung gemäß Anspruch 18, weiter umfassend eine zweite Tasche (300, 300a, 300c, 300d, 300e), und wobei die ersten und zweiten Taschen (300, 300a, 300c, 300d, 300e) miteinander verbunden sind.

21. Die medizinische Vorrichtung gemäß Anspruch 1, wobei zumindest eine der ersten und zweiten Schichten (100, 200) eine Mehrzahl von Unterschichten umfasst.

22. Die medizinische Vorrichtung gemäß Anspruch 21, wobei zumindest zwei Unterschichten ein unterschiedliches Material umfassen.

23. Die medizinische Vorrichtung gemäß Anspruch 21, wobei zumindest zwei Unterschichten von unterschiedlicher Dicke sind.

24. Die medizinische Vorrichtung gemäß Anspruch 1, weiter umfassend eine Barriere (310) zwischen den ersten und zweiten Schichten (100, 200).

25. Die medizinische Vorrichtung gemäß Anspruch 1, weiter umfassend eine dritte Schicht angeordnet über zumindest einem Teil einer der ersten und zweiten Schichten (100, 200).

26. Die medizinische Vorrichtung gemäß Anspruch 1, wobei die ersten und zweiten Schichten (100, 200) das gleiche Material umfassen.

27. Die medizinische Vorrichtung gemäß Anspruch 1, wobei die ersten und zweiten Schichten (100, 200) unterschiedliche Materialien umfassen.

28. Die medizinische Vorrichtung gemäß Anspruch 1, wobei die ersten und zweiten Schichten (100, 200) unterschiedliche Zugfestigkeiten haben.

29. Die medizinische Vorrichtung gemäß Anspruch 1, wobei die ersten und zweiten Schichten (100, 200) von unterschiedlicher Dicke sind.

30. Die medizinische Vorrichtung gemäß Anspruch 1, wobei zumindest eine der ersten und zweiten Schicht (100, 200) im Stande ist durch die Expansion des Stents (10) zerrissen zu werden.

31. Die medizinische Vorrichtung gemäß Anspruch 1, wobei zumindest ein Teil von zumindest einer der ersten und zweiten Schicht (100, 200) eine Mehrzahl von Poren umfasst.

32. Die medizinische Vorrichtung gemäß Anspruch 1, wobei zumindest eine der ersten Schicht (100) und der zweiten Schicht (200) zumindest eine vorgeformte Prägung umfasst, und wobei der geprägte Bereich üblicherweise eine geringere Zugfestigkeit hat als der Rest der Schicht.

33. Die medizinische Vorrichtung gemäß Anspruch 1, wobei zumindest eine der ersten Schicht (100) und der zweiten Schicht (200) ein selbstabdichtendes Material umfasst.

34. Die medizinische Vorrichtung gemäß Anspruch 1, wobei zumindest eine der ersten Schicht (100) und der zweiten Schicht (200) ein biologisch abbaubares Material umfasst.

35. Die medizinische Vorrichtung gemäß Anspruch 1, wobei zumindest eine der ersten Schicht (100) und der zweiten Schicht (200) im Wesentlichen flexibel ist.

36. Die medizinische Vorrichtung gemäß Anspruch 1, wobei das Therapeutikum (400, 400a, 400b) aus der ersten Tasche (300, 300a, 300c, 300d, 300e) durch zumindest eine der ersten Schicht (100) und zweiten Schicht (200) freisetzbar ist.

37. Die medizinische Vorrichtung gemäß Anspruch 1, wobei das Therapeutikum (400, 400a, 400b) aus der ersten Tasche (300, 300a, 300c, 300d, 300e) nach der Expansion des Stents (10) freisetzbar ist.

## Revendications

1. Dispositif médical pour la délivrance d'un agent thérapeutique (400, 400a, 400b) comportant :
(a) un stent (10) ayant une paroi latérale (20) comportant une pluralité d'entretoises (30, 30a, 30b) et au moins une première ouverture (40) dans la paroi latérale, la première ouverture (40) étant située entre des entretoises adjacentes (30, 30a, 30b), la paroi latérale ayant une première surface de paroi latérale (50, 60), dans lequel la première surface de paroi latérale (50, 60) est soit une surface de paroi latérale extérieure (60), qui fait face à une paroi de lumen de corps lorsque le stent est implanté, soit une surface de paroi latérale intérieure (50), qui est éloignée de la paroi de lumen de corps ;
(b) une première couche (100) disposée au-dessus d'au moins une partie de la première surface de paroi latérale (50, 60), dans lequel au moins une portion de la première couche (100) s'étend au-dessus d'une partie de la première ouverture (40) ;
(c) une seconde couche (200) disposée au-dessus d'au moins une partie de la première surface de paroi latérale (50, 60), dans lequel au moins une portion de la seconde couche (200) est disposée au-dessus de la portion de la première couche (100) qui s'étend au-dessus de la première ouverture (40),
(d) au moins une première poche (300, 300a, 300c, 300d, 300e) disposée contre au moins une portion de la première ouverture (40), au moins une portion de la première poche (300, 300a, 300c, 300d, 300e) s'étendant dans la première ouverture (40), dans lequel la poche est définie au moins en partie par une portion de la première couche (100), une portion de la seconde couche (200), et par une première liaison entre la première couche (100) et la second couche (200) ; et
(e) un agent thérapeutique (400, 400a, 400b) contenu dans la première poche (300, 300a, 300c, 300d, 300e).

2. Dispositif médical selon la revendication 1, dans lequel la première couche (100) est reliée au stent (10).

3. Dispositif médical selon la revendication 1, dans lequel la seconde couche (200) est reliée au stent (10).

4. Dispositif médical selon la revendication 1, dans lequel la première poche (300, 300a, 300c, 300d, 300e) est co-extensible avec la première ouverture (40).

5. Dispositif médical selon la revendication 1, dans lequel la première poche (300, 300a, 300c, 300d, 300e) est située dans la première ouverture (40).

6. Dispositif médical selon la revendication 1, dans lequel la première poche (300, 300a, 300c, 300d, 300e) s'étend au-delà de la première ouverture (40).

7. Dispositif médical selon la revendication 1, comportant en outre une seconde poche disposée contre la première ouverture (40).

8. Dispositif médical selon la revendication 1, dans lequel au moins une entretoise (30, 30a, 30b) comporte une surface latérale (30ₛ₁, 30ₛ₂) et la première couche (100) est disposée par dessus la surface latérale.

9. Dispositif médical selon la revendication 1, dans lequel au moins une entretoise (30, 30a, 30b) comporte une surface latérale (30ₛ₁, 30ₛ₂) et la seconde couche (100) est disposée par dessus la surface latérale.

10. Dispositif médical selon la revendication 1, dans lequel au moins une entretoise (30, 30a, 30b) comporte une surface latérale (30ₛ₁, 30ₛ₂) et les première et seconde couches (100) sont disposées par dessus la surface latérale.

11. Dispositif médical selon la revendication 1, dans lequel la paroi latérale (20) comporte en outre une seconde surface de paroi latérale (50, 60) et une troisième couche est disposée par dessus la seconde surface de paroi latérale (50, 60).

12. Dispositif médical selon la revendication 11, dans lequel la paroi latérale (20) comporte en outre une quatrième couche disposée par dessus au moins une portion de la troisième couche.

13. Dispositif médical selon la revendication 1, dans lequel le stent (10) comporte en outre une seconde ouverture, et dans lequel une seconde poche est disposée autour de la deuxième ouverture.

14. Dispositif médical selon la revendication 13, dans lequel la seconde poche est définie au moins en partie par la première couche (100) et au moins en partie par la seconde couche (200).

15. Dispositif médical selon la revendication 13, dans lequel la seconde poche est définie au moins en partie par une troisième couche et au moins en partie par une quatrième couche.

16. Dispositif médical selon la revendication 15, dans lequel la troisième couche est disposée par dessus au moins une portion de la première surface de paroi latérale (50, 60), et la quatrième couche est disposée par dessus au moins une portion de la seconde surface de paroi latérale (50, 60).

17. Dispositif médical selon la revendication 13, dans lequel la seconde poche contient un agent thérapeutique (400, 400a, 400b).

18. Dispositif médical selon la revendication 1, comportant en outre une seconde poche, et dans lequel la seconde poche contient un agent thérapeutique (400, 400a, 400b) différent de celui de la première poche (300, 300a, 300c, 300d, 300e).

19. Dispositif médical selon la revendication 18, dans lequel la seconde poche est disposée autour de la première ouverture (40).

20. Dispositif médical selon la revendication 18, comportant de plus une seconde poche (300, 300a, 300c, 300d, 300e), et dans lequel les première et seconde poches (300, 300a, 300c, 300d, 300e) sont interconnectées.

21. Dispositif médical selon la revendication 1, dans lequel au moins l'une des première et seconde couches (100, 200) comporte une pluralité de sous-couches.

22. Dispositif médical selon la revendication 21, dans lequel au moins deux sous-couches comportent un matériau différent.

23. Dispositif médical selon la revendication 21, dans lequel au moins deux sous-couches ont des épaisseurs différentes.

24. Dispositif médical selon la revendication 1, comportant en outre une séparation (310) entre les première et seconde couches (100, 200).

25. Dispositif médical selon la revendication 1, comportant en outre une troisième couche disposé par dessus au moins une portion de l'une des première et seconde couches (100, 200).

26. Dispositif médical selon la revendication 1, dans lequel les première et seconde couches (100, 200) sont réalisées dans le même matériau.

27. Dispositif médical selon la revendication 1, dans lequel les première et seconde couches (100, 200) sont réalisées dans des matériaux différents.

28. Dispositif médical selon la revendication 1, dans lequel les première et seconde couches (100, 200) ont des résistances à la rupture différentes.

29. Dispositif médical selon la revendication 21, dans lequel les première et seconde couches (100, 200) ont des épaisseurs différentes.

30. Dispositif médical selon la revendication 1, dans lequel au moins l'une des première et seconde couches (100, 200) peut se rompre par extension du stent (10).

31. Dispositif médical selon la revendication 1, dans lequel au moins une portion d'au moins une des première et seconde couches (100, 200) comporte une pluralité de pores.

32. Dispositif médical selon la revendication 1, dans lequel au moins l'une des première (100) et seconde (200) couches comporte au moins une empreinte préformée, et dans lequel la zone imprimée possède de façon générale une résistance à la rupture inférieure à celle du reste de la couche.

33. Dispositif médical selon la revendication 1, dans lequel au moins une des première (100) et seconde (200) couches comporte un matériau auto-obturant.

34. Dispositif médical selon la revendication 1, dans lequel au moins une des première (100) et seconde (200) couches comporte un matériau biodégradable.

35. Dispositif médical selon la revendication 1, dans lequel au moins une des première (100) et seconde (200) couches sont sensiblement flexibles.

36. Dispositif médical selon la revendication 1, dans lequel l'agent thérapeutique (400, 400a, 400b) peut être libérer de la première poche (300, 300a, 300c, 300d, 300e) à travers au moins l'une des première (100) et seconde (200) couches.

37. Dispositif médical selon la revendication 1, dans lequel l'agent thérapeutique (400, 400a, 400b) peut être libérer de la première poche (300, 300a, 300c, 300d, 300e) après extension du stent (10).
